# EUROPÄISCHE PATENTSCHRIFT

(11) **EP 2 720 739 B1**
(45) Veröffentlichungstag und Bekanntmachung des Hinweises auf die Patenterteilung: **30.08.2017**
(21) Anmeldenummer: 12728452.9
(22) Anmeldetag: 12.06.2012
(51) Int. Cl.: A61M 5/00, A61J 1/06, A61J 1/20, B65B 3/04

(54) **TRANSPORT- UND TRANSFERBEHÄLTNIS FÜR EIN FLUIDES MEDIUM**
TRANSPORT AND TRANSFER CONTAINER FOR A FLUID MEDIUM
RECIPIENT DE TRANSPORT ET DE TRANSFERT POUR UN MEDIUM FLUIDIQUE

(30) Priorität: 16.06.2011 EP 11170210
(43) Veröffentlichungstag der Anmeldung: 23.04.2014
(73) Patentinhaber: Roche Diabetes Care GmbH, 68305 Mannheim (DE); F. Hoffmann-La Roche AG, 4070 Basel (CH)
(72) Erfinder: ARNITZ, Theo, 68753 Waghaeusel (DE); HARTIG, Stefan, 77855 Achern (DE)
(74) Vertreter: Herzog, Fiesser & Partner Patentanwälte PartG mbB
(86) Internationale Anmeldenummer: PCT/EP2012/061079
(87) Internationale Veröffentlichungsnummer: WO 2012/171898

(56) Entgegenhaltungen:
- EP-A1- 2 258 426
- WO-A2-2008/083209
- US-A- 4 201 207
- US-A- 4 765 512

## Beschreibung

### Gebiet der Erfindung

Die Erfindung betrifft ein Verfahren zur Herstellung eines Transport- und Transferbehältnisses für mindestens ein fluides Medium. Weiterhin betrifft die Erfindung ein Bereitstellungsverfahren zur Bereitstellung mindestens eines fluiden Mediums sowie ein Transport- und Transferbehältnis für mindestens ein fluides Medium. Derartige Verfahren und Vorrichtungen werden insbesondere im Bereich der Medizintechnik eingesetzt, um fluide Medien, insbesondere Flüssigkeiten, steril über mehrere Wochen oder Monate lagern zu können und anschließend auf einfache und zuverlässige Weise bereitstellen zu können, beispielsweise an eine Infusionseinrichtung, eine Injektionsvorrichtung oder eine Medikationsvorrichtung, beispielsweise eine Insulinpumpe oder eine andere Art von Medikationspumpe.

### Stand der Technik

Aus dem Stand der Technik ist eine Vielzahl von Behältnissen für fluide Medien, insbesondere Flüssigkeiten, bekannt, die grundsätzlich für eine Lagerung, einen Transport und einen Transfer von fluiden Medien sowie gegebenenfalls für eine Injektion derartiger fluider Medien eingesetzt werden können.

So beschreibt beispielsweise US 7,670,314 B2 eine Injektionsvorrichtung, welche beispielsweise für eine Selbstmedikation geeignet ist. Diese umfasst ein Gehäuse, als Basis zum Aufsetzen auf eine Hautoberfläche eines Patienten. In der Basis ist eine Injektionsnadel aufgenommen, welche gleichzeitig eine Hautoberfläche eines Patienten und ein Septum eines Flüssigkeitsreservoirs durchdringen kann. Weiterhin ist eine manuell bedienbare Druckfeder vorgesehen, welche einen Druck auf eine injizierbare Flüssigkeitszusammensetzung innerhalb des Reservoirs ausübt. Nachteilig an dem in US 7,670,314 B2 beschrieben System ist jedoch insgesamt, dass die Beaufschlagung des Behältnisses mit dem Überdruck, welche durch einen Patienten oder medizinisches Fachpersonal unmittelbar während der Injektion erfolgt, stets mit der Injektion selbst gekoppelt ist. Hierdurch muss der Patient oder das medizinische Fachpersonal selbst die zur Druckbeaufschlagung und zur Perforation erforderliche Energie aufbringen. Eine weitere Problematik ergibt sich daraus, dass in der Flüssigkeit in der Regel Gasblasen vorhanden sind, welche bei einer gleichzeitigen Druckbeaufschlagung und Injektion möglicherweise in das Körpergewebe des Patienten geraten können.

Aus US 5,858,001 ist eine Injektionsvorrichtung zum Einsatz auf einer Hautoberfläche eines Patienten bekannt. Diese umfasst eine Basis, welche auf die Hautoberfläche aufgeklebt werden kann. Weiterhin ist eine Kartusche vorgesehen, in welcher ein Medikament aufgenommen ist. Bei einem Aufsetzen auf die Haut wird durch einen entsprechenden Mechanismus gleichzeitig ein Druckanstieg in der Kartusche und eine Herstellung einer Fluidverbindung zu einer Injektionsnadel hergestellt. Nachteilig an dieser Konstruktion ist jedoch wiederum, dass sich in der Kartusche Gasblasen bilden können, welche sich aufgrund der dargestellten Gleichzeitigkeit des Druckanstiegs und der Penetration der Hautoberfläche durch die Injektionsnadel nicht auflösen können, sodass diese ins Körpergewebe gelangen können. Ein weiterer Nachteil besteht in der Komplexität des beschriebenen Systems, da die Gleichzeitigkeit der beschriebenen Vorgänge ein Zusammenwirken einer Mehrzahl von Komponenten erforderlich macht, insbesondere ein Zusammenwirken mehrerer Bauteile. Weiterhin kann ein Nachteil der beschriebenen Konstruktion darin bestehen, dass eine Bewegung eines Druckbeaufschlagungssystems parallel zu einer Hautoberfläche in einem Zustand erfolgt, in welchem eine Injektionsnadel bereits die Hautoberfläche penetriert hat, was zu einer erhöhten Schmerzbelastung führen kann. Ein weiterer Nachteil besteht, wie auch bei der zuvor beschrieben US 7,670,314 B2, auch bei der US 5,858,001 darin, dass eine Dichtigkeitskontrolle des Systems und der einzelnen Systemkomponenten erst während der Benutzung möglich ist. Sollte sich während der Benutzung jedoch herausstellen, dass aufgrund eines fehlerhaften Zusammenwirkens einzelner Systemkomponenten eine Undichtigkeit besteht, so kann während des Injektionsvorgangs ein Austreten von Flüssigkeit erfolgen, ohne dass der Injektionsvorgang noch kontrolliert abgebrochen oder mit einer definierten Injektionsmenge wiederholt werden kann.

Auch aus US 7,214,221 B2, aus US 7,252,651 B2, aus US 7,470,258 B2, WO 2009/119496 A1, US 2010/0262077 A1 und US 2004/0133159 A1 sind Injektionssysteme bekannt, bei welchen eine mechanische Druckbeaufschlagung während eines Injektionsvorgangs erfolgen kann. Auch diese Systeme weisen jedoch die oben beschriebenen Nachteile auf, insbesondere die Nachteile einer Gasblasenbildung sowie den Nachteil, dass eine Dichtigkeitskontrolle in der Regel erst während einer Verwendung der beschriebenen Systeme erfolgt. Weiterhin sind die beschriebenen Systeme teilweise technisch äußerst komplex. Weitere Nachteile bestehen darin, dass ein Wechsel der Fluidbehältnisse in der Regel nur schwer oder unter Eintrag von Luft möglich ist.

In WO 2008/083209 A2 werden zahlreiche verschiedene Ausführungsbeispiele einer Medikationsvorrichtung zum Applizieren einer unter Druck stehenden Medikamentenflüssigkeit beschrieben. Allgemein beschreibt dieses Dokument beispielsweise die Verwendung eines Flüssigkeitsreservoirs in Form eines Beutels, der in einem Gehäuse aufgenommen ist. Das Gehäuse umfasst ein Durchstoßelement, welches eine Beutelwand des Reservoirs durchstoßen kann. Vor einer Applikation des Medikaments wird das Flüssigkeitsreservoir unter Druck gesetzt, und die Wand des Reservoirs wird durchstoßen. Dieses Durchstoßen kann auf verschiedene Weisen erfolgen, beispielsweise automatisch oder durch ein entsprechendes Aufpressen auf eine Hautoberfläche. Darüber werden in verschiedenen Ausführungsbeispielen eine sterile Umverpackung, verschiedene Zeitpunkte der Druckbeaufschlagung, verschiedene Arten eines Druckbeaufschlagungsmechanismus, eine Druckbeaufschlagung bei Aufschrauben eines Deckels und verschiedene Sicherheitsmechanismen offenbart.

### Aufgabe der Erfindung

Es ist daher eine Aufgabe der vorliegenden Erfindung, ein Transport- und Transferbehältnis für ein fluides Medium sowie ein entsprechendes Herstellungsverfahren vorzuschlagen, welche die Nachteile bekannter Transport- und Transferbehältnisse und Herstellungsverfahren zumindest weitgehend vermeiden. Insbesondere soll das Transport- und Transferbehältnis auf einfache Weise ausgestaltet sein, soll auch für große Flüssigkeitsmengen einsetzbar sein und soll eingerichtet sein, um bei der Befüllung entstehende Luftblasen vorzugsweise vollständig zu entfernen.

### Offenbarung der Erfindung

Diese Aufgabe wird gelöst durch die Erfindung mit den Merkmalen der unabhängigen Patentansprüche. Vorteilhafte Weiterbildungen der Erfindungen, welche einzeln oder in Kombination realisierbar sein, sind in den abhängigen Ansprüchen dargestellt.

Im Rahmen der vorliegenden Erfindung werden die Begriffe "umfasst", "enthält" und "weist auf" sowie entsprechende grammatikalische Abwandlungen davon derart verstanden, dass diese Begriffe sowohl die Möglichkeit einer abschließenden Aufzählung von Komponenten im Sinne von "besteht aus" beinhalten können als auch die Möglichkeit einer nicht-abschließenden Aufzählung. So können beispielsweise die Ausdrücke "A weist B auf", "A enthält B" und "A umfasst B" derart verstanden werden, dass A entweder ausschließlich aus B besteht oder dass A neben B mindestens eine weitere Komponente enthält.

In einem ersten Aspekt der vorliegenden Erfindung wird ein Herstellungsverfahren zur Herstellung eines Transport- und Transferbehältnisses für mindestens ein fluides Medium vorgeschlagen. Unter einem Transport- und Transferbehältnis ist dabei allgemein ein Behältnis zu verstehen, mittels dessen ein fluides Medium, insbesondere eine Flüssigkeit und besonders bevorzugt ein flüssiges Therpeutikum und/oder Diagnostikum, transportiert und bereitgestellt werden kann. Der Transport und/oder die Bereitstellung kann insbesondere steril erfolgen. Der Transport kann beispielsweise von einem Hersteller zu einem Zwischenhändler, von einem Zwischenhändler zu einem Händler oder von einem Händler zu einem Endkunden oder Anwender erfolgen und kann optional auch beispielsweise eine Lagerung beinhalten. Unter einem Transfer ist im Rahmen der vorliegenden Erfindung ein Vorgang zu verstehen, bei welchem das fluide Medium aus dem Transport- und Transferbehältnis in eine weitere Vorrichtung, beispielsweise einen weiteren Behälter und/oder eine Fluidvorrichtung, oder in anderer Weise aus dem Transport- und Transferbehältnis heraus befördert wird, sodass das Transport- und Transferbehältnis vollständig oder teilweise entleert wird.

Das fluide Medium kann, wie oben ausgeführt, insbesondere mindestens eine Flüssigkeit umfassen. Insbesondere kann es sich bei dieser Flüssigkeit um mindestens eine sterile Flüssigkeit handeln, also eine Flüssigkeit, welche unter keimarmen Bedingungen erzeugt und/oder abgefüllt und/oder gelagert und/oder verwendet wird.

Das Verfahren umfasst die folgenden Verfahrensschritte, welche vorzugsweise, jedoch nicht notwendigerweise, in der nachfolgend dargestellten Reihenfolge durchgeführt werden. Weiterhin können auch einzelne oder mehrere der genannten Verfahrensschritte wiederholt, zeitlich parallel oder zeitlich überlappend durchgeführt werden. Weiterhin können zusätzliche, im Folgenden nicht genannte Verfahrensschritte vorgesehen sein.
a) Bereitstellen mindestens eines Behälters zur Aufnahme des fluiden Mediums;
b) Bereitstellen mindestens einer integrierten Energieeinheit, wobei die Energieeinheit eingerichtet ist, um bei einer Verwendung des Transport- und Transferbehältnisses eine - vorzugsweise vollständige - Entleerung des fluiden Mediums aus dem Behälter zu bewirken;
c) Einbringen des fluiden Mediums in den Behälter und Verschließen des Behälters mit einem verschiebbaren Stopfen;
d) Beaufschlagen des fluiden Mediums mit einem Überdruck, unter Verwendung der Energieeinheit, wobei die Energieeinheit mindestens ein Federelement umfasst wobei der Überdruck bis zu einer Entnahme des fluiden Mediums aufrechtgehalten wird; und
e) Verpacken zumindest des Behälters in dem mit Überdruck beaufschlagten Zustand in mindestens einer Verpackung.

Das Transport- und Transferbehältnis umfasst also mindestens einen Behälter zur Aufnahme des fluiden Mediums. Unter einem Behälter ist dabei allgemein eine Vorrichtung zu verstehen, welche mindestens einen Innenraum zur Aufnahme des fluiden Mediums umfasst sowie mindestens eine Behälterwand, welche das fluide Medium in dem Innenraum zurückhält. Beispielsweise kann der Behälter ganz oder teilweise aus einem Material hergestellt sein, ausgewählt aus der Gruppe bestehend aus einem Glasmaterial und einem Kunststoffmaterial. Unter einer Energieeinheit ist im Rahmen der vorliegenden Erfindung eine Vorrichtung zu verstehen, welche eingerichtet ist, um eine vollständige oder teilweise Entleerung des fluiden Mediums aus dem Behälter zu bewirken. Zu diesem Zweck kann die Energieeinheit beispielsweise eingerichtet sein, um mechanisch einen Druck auf das fluide Medium auszuüben. Zu diesem Zweck kann die Energieeinheit beispielsweise eine oder mehrere Vorrichtungen umfassen, welche eingerichtet sind, um ein Volumen des Innenraums des Behälters zu verkleinern, beispielsweise einen in dem Innenraum beweglichen Kolben und/oder Stopfen. Darüber hinaus kann die Energieeinheit mindestens einen Energiespeicher umfassen, welcher eingerichtet ist, um eine Energiemenge, vorzugsweise eine mechanische Energiemenge, zu speichern, beispielsweise mindestens einen Federspeicher. Beispielsweise kann der Energiespeicher mit der Vorrichtung gekoppelt sein, welcher eingerichtet ist, um den Druck auf das fluide Medium auszuüben. Beispielsweise kann die Energieeinheit mindestens ein mechanisches Federelement umfassen, welches mit mindestens einem Kolben und/oder Stopfen der Enerieeinheit gekoppelt ist. Beispielsweise kann es sich bei diesem Federelement um mindestens eine Schraubendruckfeder, eine Sprungfeder und/oder Spiralfeder handeln. Die in der Energieeinheit gespeicherte mechanische Energiemenge ist derart eingerichtet, dass diese für eine - vorzugsweise vollständige - Entleerung des fluiden Mediums aus dem Behälter ausreichend ist, vorzugsweise auch über eine Lagerdauer von mindestens zwei Wochen, mindestens vier Wochen, mindestens zwei Monaten oder sogar mindestens vier Monaten und insbesondere sogar mindestens einem Jahr oder mehrere Jahre oder für mindestens die so genannte Lagerlebensdauer (shelf lifetime) des Produkts.

Unter einer Verwendung des Transport- und Transferbehältnisses ist im Rahmen der vorliegenden Erfindung ein Vorgang zu verstehen, bei welchem das fluide Medium bestimmungsgemäß aus dem Behälter des Transport- und Transferbehältnisses heraustransferiert wird. Die Begriffe der Verwendung, der bestimmungsgemäßen Verwendung, der vollständigen oder teilweisen Entleerung und des Transfers des fluiden Mediums aus dem Behälter heraus werden im Folgenden im Wesentlichen synonym verwendet. Dieser Transfer kann insbesondere unter Verwendung der Energieeinheit erfolgen, beispielsweise indem der mechanische Energiespeicher der Energieeinheit entladen wird, die Energieeinheit einen Druck auf das fluide Medium ausübt und das fluide Medium, beispielsweise nach Herstellung einer Fluidverbindung, aus dem Behälter heraus transferiert wird.

Unter einem Einbringen des fluiden Mediums in den Behälter wird allgemein im Rahmen der vorliegenden Erfindung ein Abfüllen des fluiden Mediums verstanden, bei welchem der Behälter vollständig oder teilweise mit dem mindestens einen fluiden Medium befüllt wird. Dieses Einbringen kann beispielsweise automatisiert erfolgen, beispielsweise mittels einer entsprechenden Abfüllanlage.

Unter einem Verschließen des Behälters wird ein Vorgang verstanden, bei welchem der Behälter verschlossen wird. Dies kann beispielsweise durch mindestens ein Verschlusselement erfolgen, vorzugsweise durch mindestens einen Stopfen, insbesondere einen perforierbaren Stopfen und besonders bevorzugt durch mindestens einen perforierbaren, verschieblich gelagerten Stopfen. Das Verschlusselement kann selbst als Bestandteil des Behälters angesehen werden. Das Verschließen erfolgt vorzugsweise derart, dass das fluide Medium in einem Innenraum des Behälters zurückgehalten wird und diesen Innenraum zumindest ohne nachträgliche Herstellung einer Fluidverbindung zu dem Innenraum nicht mehr verlassen kann.

Unter einer Beaufschlagung des fluiden Mediums mit einem Überdruck wird im Rahmen der vorliegenden Erfindung ein Vorgang verstanden, nach dessen Durchführung das fluide Medium in dem Behälter einen Druck aufweist, welcher über dem Umgebungsdruck liegt, also vorzugsweise oberhalb eines Normaldrucks, beispielsweise einen Druck von mindestens 1,2 bar, insbesondere mindestens 1,5 bar, vorzugsweise mindestens 2 bar. Die Druckbeaufschlagung erfolgt vorzugsweise durch einen Hersteller des Transport- und Transferbehältnisses, also beispielsweise im Rahmen eines Herstellungsverfahrens in einer Anlage oder in einem Werk, in welchem die Verfahrensschritte a) bis d) durchgeführt werden.

Unter einer Aufrechterhaltung des Überdrucks bis zu einer Entnahme des fluiden Mediums wird ein Vorgang verstanden, bei welchem das fluide Medium in dem Behälter des Transport- und Transferbehältnisses bis zu einem bestimmungsgemäßen Transfer des fluiden Mediums aus dem Behälter heraus einen Druck oberhalb eines Umgebungsdrucks aufweist, vorzugsweise einen Druck von mindestens 1,2 bar, besonders bevorzugt von mindestens 1,5 bar und insbesondere von mindestens 2 bar. Beispielsweise kann bei der Herstellung anfänglich das fluide Medium in dem Behälter mit einem Anfangsdruck oberhalb des Umgebungsdrucks beaufschlagt werden, wobei der Überdruck bis zur Entnahme des fluiden Mediums derart aufrechtgehalten wird, dass der Überdruck bis zur Entnahme des fluiden Mediums auf nicht weniger als 50% des Anfangsdrucks abgefallen ist, vorzugsweise auf nicht weniger als 70%, besonders bevorzugt auf nicht weniger als 80% und besonders bevorzugt auf nicht weniger als 90% des Anfangsdrucks. Dies kann beispielsweise durch eine entsprechende Ausgestaltung der Energieeinheit erfolgen, beispielsweise indem ein entsprechender mechanischer Energiespeicher gewählt wird, welcher über mindestens zwei Wochen, vorzugsweise über mindestens vier Wochen, mindestens einen Monat, mindestens zwei Monate, mindestens vier Monate, mindestens ein Jahr oder mehrere Jahre oder über mindestens die gesamte Lagerlebensdauer des Produkts in einem derart gespannten Zustand verbleiben kann, dass die oben beschriebenen Druckbedingungen erfüllt sind. Weiterhin kann auch ein entsprechend dichter Behälter verwendet werden.

Das Herstellungsverfahren umfasst, wie oben ausgeführt, folgenden Verfahrensschritt :
e) Verpacken zumindest des Behälters des Transport- und Transferbehältnisses in dem mit dem Überdruck beaufschlagten Zustand in mindestens einer Verpackung.

Dieser Verfahrensschritt e) kann insbesondere nach Durchführung des Verfahrensschritts d) durchgeführt werden. Unter einem Verpacken wird dabei im Rahmen der vorliegenden Erfindung ein Prozess verstanden, bei welchem zumindest ein Teil des Transport- und Transferbehältnisses, welches zumindest den Behälter umfasst, vollständig oder zumindest teilweise von der mindestens einen Verpackung umhüllt wird. Neben dem Behälter können ein oder mehrere weitere Elemente des Transport- und Transferbehältnisses vollständig oder teilweise von der Verpackung umhüllt werden. Beispielsweise können zumindest der Behälter und die Energieeinheit ganz oder teilweise verpackt werden, vorzugsweise in einem Zustand, in welchem das fluide Medium durch die Energieeinheit mit dem Überdruck beaufschlagt ist. So können beispielsweise der Behälter und die Energieeinheit eine Einheit bilden, welche in der Verpackung aufgenommen ist. Die Verpackung kann eingerichtet sein, um das Transport- und Transferbehältnis gegenüber Umwelteinflüssen wie beispielsweise Luft, Feuchtigkeit, mechanischen Einflüssen oder Kombinationen der genannten Umwelteinflüsse und/oder anderer Umwelteinflüsse zu schützen. Beispielsweise kann diese Verpackung eine Sterilverpackung umfassen, also eine keimdichte Verpackung, und/oder eine Verpackung, welche undurchlässig ist gegenüber Luft. Beispielsweise kann die Verpackung mindestens eine Kunststoffverpackung umfassen. So können der Behälter sowie optional ein oder mehrere weitere Teile des Transport- und Transferbehältnisses insbesondere in eine Kunststoffverpackung, beispielsweise eine Folienverpackung, eingeschweißt werden, beispielsweise in einen so genannten Blister Pack.

Die Verpackung kann weiterhin als integralen oder separaten Bestandteil mindestens eine Kennzeichnung enthalten, beispielsweise eine Kennzeichnung des Transport- und Transferbehältnisses und/oder eine Kennzeichnung des fluiden Mediums. Beispielsweise kann eine produktkonforme Kennzeichnung enthalten sein. Diese Kennzeichnung kann beispielsweise mindestens eine Information umfassen, welche das fluide Medium und/oder dessen Eigenschaften und/oder dessen Nutzung kennzeichnet. Beispielsweise kann die Kennzeichnung mindestens eine durch einen Menschen und/oder eine durch eine Maschine lesbare Information umfassen. Die mindestens eine Information kann beispielsweise ausgewählt sein aus der Gruppe bestehend aus: einer Identität des fluiden Mediums, insbesondere einer Art und/oder einer Konzentration mindestens eines Wirkstoffs in dem fluiden Medium; einer Herstellerangabe; einer Chargennummer des fluiden Mediums; einer Verwendung des fluiden Mediums; einem Herstelldatum des fluiden Mediums; einem Verfallsdatum und/oder Aufbrauchdatum des fluiden Mediums. Die Kennzeichnung kann beispielsweise auf der Verpackung aufgebracht und/oder in die Verpackung eingebracht sein. Beispielsweise kann die Kennzeichnung mindestens einen Aufdruck auf der Verpackung umfassen und/oder mindestens ein Kennzeichnungsetikett, welches auf die Verpackung aufgeklebt ist. Auch andere Arten der Kennzeichnung sind möglich.

Das Herstellungsverfahren kann weiterhin insbesondere derart durchgeführt werden, dass nach Durchführung des Verfahrensschritts d) und vor einer Verwendung des Transport- und Transferbehältnisses eine Lagerung des Transport- und Transferbehältnisses für eine Zeitdauer von mindestens zwei Wochen, vorzugsweise von mindestens drei Wochen, erfolgt, insbesondere in einem mit dem Überdruck beaufschlagten und verpackten Zustand. Die Lagerung kann also insbesondere auch nach Durchführung des Verfahrensschritts e) erfolgen, also in verpacktem Zustand des Transport- und Transferbehältnisses.

Unter einer Verwendung ist dabei allgemein ein Vorgang zu verstehen, bei welchem das fluide Medium aus dem Innenraum des Behälters des Transport- und Transferbehältnisses bereitgestellt wird, beispielsweise indem eine Fluidverbindung zwischen dem Innenraum und einem Außenraum und/oder einem Gefäß hergestellt wird, durch welche das fluide Medium aus dem Behälter heraus transferiert wird. Die Lagerung kann beispielsweise unmittelbar beim Hersteller erfolgen oder auch bei einem Zwischenhändler oder Händler, bevor das Transport- und Transferbehältnis zu einem Benutzer transportiert wird, beispielsweise zu einem Arzt, Pflegepersonal oder einem Patienten. Dementsprechend kann die Beaufschlagung des fluiden Mediums mit dem Überdruck insbesondere herstellerseitig erfolgen, also nicht durch einen Anwender, der das Transport- und Transferbehältnis bestimmungsgemäß verwendet.

Unter einer Lagerung ist allgemein im Rahmen der vorliegenden Erfindung ein Vorgang zu verstehen, bei welchem das Transport- und Transferbehältnis nicht verwendet wird und vorzugsweise ruht. Die Lagerung kann beispielsweise beim Hersteller und/oder auch bei einem Verteiler und/oder bei einem Händler und/oder bei einem Zwischenhändler erfolgen. Die Lagerung kann unter kontrollierten Bedingungen erfolgen, bei welchen beispielsweise eine Umgebungstemperatur in einen vorgegebenen Bereich eingestellt wird.

Im Verfahrensschritt c) kann weiterhin zunächst mindestens eine Gasblase in dem Behälter erzeugt werden. Verfahrensschritt d) kann dann insbesondere derart durchgeführt werden, dass die Gasblase nach einer Wartezeit, beispielsweise einer Wartezeit von mindestens einer Stunde und besonders bevorzugt von mindestens zwei Stunden, beispielsweise nach einer Wartezeit von einer Stunde bis einer Woche und besonders bevorzugt nach einer Wartezeit von zwei Stunden bis einer Woche, vollständig verschwindet, sodass ein gasblasenfreies Transport- und Transferbehältnis entsteht. Beispielsweise kann Verfahrensschritt d) derart durchgeführt werden, dass der Überdruck, mit welchem das fluide Medium beaufschlagt wird, derart hoch gewählt wird, dass die Gasblase sich zumindest nach der genannten Wartezeit, welche insbesondere herstellerseitig verbracht werden kann, vollständig auflöst. Dies kann beispielsweise durch eine entsprechende Dimensionierung des mechanischen Energiespeichers der Energieeinheit erfolgen, sodass beispielsweise die Druckbeaufschlagung mit einem entsprechenden Überdruck erfolgen kann.

Die Verfahrensschritte a) - e) können, wie oben ausgeführt, insbesondere vor einem Vertrieb des Transport- und Transferbehältnisses an einen Kunden, insbesondere an einen Endkunden, durchgeführt werden. Unter einem Kunden ist dabei allgemein eine Person zu verstehen, welche das Transport- und Transferbehältnis bestimmungsgemäß verwendet. Wie oben ausgeführt, kann es sich bei diesem Kunden insbesondere um einen Arzt oder ein Krankenhaus, eine Krankenpflegeeinrichtung, eine Altenpflegeeinrichtung oder auch einen Patienten selbst handeln.

Weitere bevorzugte Ausgestaltungen betreffen den Verfahrensschritt d) und die Druckbeaufschlagung. So kann in dem Verfahrensschritt d) insbesondere mindestens ein Federelement des Transport- und Transferbehältnisses, insbesondere mindestens ein Federelement der Energieeinheit, beispielsweise mindestens eines Energiespeichers der Energieeinheit, gespannt werden. Unter einem Spannen ist dabei ein zumindest teilweise reversibles Speichern mechanischer Energie in dem Federelement zu verstehen, beispielsweise indem das Federelement gedehnt oder komprimiert wird.

Das Federelement kann insbesondere auf mindestens einen verschiebbaren Abschnitt des Behälters einwirken, beispielsweise auf mindestens einen Stopfen und besonders bevorzugt auf mindestens einen perforierbaren Stopfen, beispielsweise auf einen Stopfen, welcher in dem Behälter derart beweglich ist, dass dieser einen Innenraum des Behälters in seinem Volumen beeinflussen kann. Beispielsweise kann der Behälter mindestens eine röhrenförmige Behälterwand umfassen, beispielsweise einen vollständig oder teilweise aus Glas und/oder Kunststoff hergestellten röhrenförmigen Behälter, in welchem der mindestens eine Stopfen eingebracht ist, beispielsweise derart, dass dieser parallel zu einer Achse des röhrenförmigen Behälters bewegbar ist.

Unter einem Stopfen ist im Rahmen der vorliegenden Erfindung ein Dichtelement zu verstehen, welches eingerichtet ist, um den Behälter zu verschließen, beispielsweise reversibel. Beispielsweise kann der Stopfen eine Zylinderform aufweisen oder im Wesentlichen zylinderförmig ausgestaltet sein, beispielsweise mit einem runden, ovalen oder polygonalen Querschnitt in einer Schnittrichtung senkrecht zu einer Achse des Behälters, beispielsweise einer Rotationsachse des Behälters. Ein Außendurchmesser des Stopfens kann beispielsweise an einen Innendurchmesser eines Innenraums des Behälters angepasst sein, beispielsweise an einen Durchmesser oder Äquivalentdurchmesser eines röhrenförmigen Behälters. Der Stopfen kann vorzugsweise ganz oder teilweise beweglich in dem Behälter gelagert sein. Unter einem perforierbaren Stopfen ist allgemein im Rahmen der vorliegenden Erfindung ein Stopfen zu verstehen, welcher mittels mindestens eines Nadelelements, beispielsweise mittels einer Kanüle, einer Hohlnadel oder einer Vollnadel, unter Aufwendung üblicher Kräfte derart perforierbar ist, dass, wie unten noch näher ausgeführt wird, eine Fluidverbindung zu dem Innenraum des Behälters durch den Stopfen hindurch herstellbar ist. Beispielsweise kann der Stopfen derart ausgestaltet sein, dass dieser manuell perforierbar ist. Beispielsweise kann der Stopfen zu diesem Zweck aus einem Kunststoffmaterial hergestellt sein, insbesondere einem Elastomermaterial und besonders bevorzugt einem Gummimaterial. Der Stopfen kann beispielsweise mindestens einen perforierbaren Bereich aufweisen, welcher vorzugsweise eine Dicke von nicht mehr als 5 mm, insbesondere eine Dicke von nicht mehr als 3 mm und besonders bevorzugt eine Dicke von nicht mehr als 2 mm aufweist, welche bei der Perforation von dem Nadelelement durchbohrbar ist.

Der Verfahrensschritt d) kann insbesondere derart durchgeführt werden, dass der Überdruck mindestens ein zu einem Transfer des fluiden Mediums aus dem Behälter heraus erforderliches Druckniveau einnimmt. Beispielsweise kann dies dadurch erfolgen, dass das Transport- und Transferbehältnis für eine bestimmte Art des Transfers eingerichtet wird, beispielsweise mittels einer entsprechenden Perforationsvorrichtung. Beispielsweise kann dabei ein entsprechender Transferquerschnitt und eine entsprechende Transferlänge hergestellt werden, wobei das Druckniveau nach Durchführung des Verfahrensschritts d) derart eingestellt wird, dass, beispielsweise unter Berücksichtigung entsprechender Strömungseigenschaften, der Druck für einen Transfer des fluiden Mediums durch diesen Transferweg ausreichend ist. Dieser Druck kann beispielsweise analytisch, empirisch oder semiempirisch bestimmt werden, beispielsweise unter Berücksichtigung des Gesetzes von Hagen-Poiseuille.

Der Behälter kann insbesondere derart ausgestaltet sein, dass das fluide Medium nach Durchführung des Verfahrensschritts d) und vor der Entnahme des fluiden Mediums, also vor einem bestimmungsgemäßen Transfer des fluiden Mediums aus dem Innenraum des Behälters heraus, ausschließlich mit einer Wand des Behälters und einem perforierbaren Abschnitt des Behälters, insbesondere einem perforierbaren Stopfen, in Kontakt steht. Beispielsweise kann der Behälter, wie oben ausgeführt, röhrenförmig ausgestaltet sein und kann eine Behälterwand sowie den perforierbaren Stopfen umfassen, sodass das fluide Medium ausschließlich mit diesen Elementen Kontakt aufweist. Dies hat insbesondere Vorteile hinsichtlich einer Produktstabilität und/oder Fluidstabilität über einen längeren Lagerzeitraum. Beispielsweise kann der röhrenförmige Behälter an einem Ende sowie umfangsseitig durch die Behälterwand verschlossen sein, beispielsweise eine Behälterwand aus einem Glasmaterial und/oder einem Kunststoffmaterial, sowie weiterhin durch genau einen Stopfen, vorzugsweise genau einen perforierbaren Stopfen, vorzugsweise einen perforierbaren Stopfen, welcher in dem röhrenförmigen Behälter verschiebbar gelagert ist. Beispielsweise kann der perforierbare Stopfen somit einerseits Bestandteil der Energieeinheit sein und eingerichtet sein, um im Verfahrensschritt d) das fluide Medium mit dem Überdruck zu beaufschlagen, und andererseits für eine Perforierung eingerichtet sein, also zur Herstellung einer Fluidverbindung zwischen einem Außenraum und dem Innenraum des Behälters.

Der Behälter kann insbesondere nach Durchführung des Verfahrensschritts d), beispielswese zwischen einer Durchführung des Verfahrensschritts d) und einer bestimmungsgemäßen Entnahme des fluiden Mediums, ohne eine Fluidverbindung zwischen einem das fluide Medium aufnehmenden Innenraum des Behälters und einer Umgebung des Behälters sein. So kann der Behälter beispielsweise ohne Ventile, verschließbare Zu- und/oder Abflüsse oder ähnliche Fluidverbindungen ausgestaltet sein. So kann der Behälter insbesondere derart ausgestaltet sein, dass eine Fluidverbindung, zumindest ohne Zerstörung des Behälters oder Teilen desselben, ausschließlich durch eine Perforation mindestens eines perforierbaren Abschnitts des Behälters, beispielsweise eine Perforation eines perforierbaren Stopfens, herstellbar ist. Beispielsweise kann der Behälter mindestens einen perforierbaren Abschnitt aufweisen, durch welchen hindurch eine Entnahme des fluiden Mediums nach einer Perforation des perforierbaren Abschnitts erfolgen kann, beispielsweise mindestens einen perforierbaren Stopfen.

Der Behälter und vorzugsweise das gesamte Transport- und Transferbehältnis kann insbesondere frei von elektrischen Komponenten ausgestaltet sein. So kann insbesondere die oben beschrieben Energieeinheit beispielsweise ganz oder teilweise als mechanische Energieeinheit ausgestaltet sein, beispielsweise als ein beweglicher Stopfen, welcher mit einem mechanischen Energiespeicher verbunden ist, beispielsweise mindestens einem Federelement, sodass über den mindestens einen beweglichen Stopfen, vorzugsweise über genau einen beweglichen Stopfen, das fluide Medium mit dem Überdruck beaufschlagbar ist. Allgemein kann der bewegliche Stopfen beispielsweise mit einem Energiespeicher gekoppelt sein, welcher ausgewählt ist aus der Gruppe bestehend aus: einem mechanischen Energiespeicher, insbesondere einem Federelement; einem chemischen Energiespeicher; einem physikalischen Energiespeicher, insbesondere einem Flüssiggas und/oder einem Medium, welches mindestens einen Phasenübergang durchführen kann, beispielsweise einem Flüssiggas. Auch andere Ausgestaltungen sind denkbar.

Das Transport- und Transferbehältnis und/oder das Herstellungsverfahren zur Herstellung desselben können insbesondere so ausgestaltet sein, dass eine separate Dichtheitskontrolle während und/oder nach der Herstellung entbehrlich ist und entfallen kann. So kann eine Undichtigkeit allein durch ein Austreten des fluiden Mediums während oder nach Durchführung des Verfahrensschritts d) erkannt werden, beispielsweise innerhalb eines Zeitraums von 0 s bis 60 s nach der Druckbeaufschlagung. Damit unterscheidet sich das vorgeschlagene Verfahren vorzugsweise von Transport- und Transferbehältnissen, bei welchen die Druckbeaufschlagung erst während des Transfers des fluiden Mediums aus dem Behälter heraus erfolgt. Das vorgeschlagene Verfahren kann somit beispielsweise eine optische Kontrolle umfassen, bei welcher festgestellt wird, ob während oder nach der Druckbeaufschlagung ein Austritt des fluiden Mediums erfolgt. Das Herstellungsverfahren kann beispielsweise derart durchgeführt werden, dass nach Durchführung des Verfahrensschritts eine Dichtheitskontrolle, insbesondere eine optische Dichtheitskontrolle, durchgeführt wird, insbesondere um einen Austritt des fluiden Mediums zu detektieren. Beispielsweise kann diese Dichtheitskontrolle innerhalb eines Zeitrahmens von maximal einem Tag, vorzugsweise innerhalb eines Zeitrahmens von maximal 10 Stunden und besonders bevorzugt von maximal 5 Stunden nach einem Beginn der Durchführung des Verfahrensschritts d) durchgeführt werden. Unter einer Dichtheitskontrolle ist allgemein ein Vorgang zu verstehen, bei welchem ein nicht-bestimmungsgemäßer Austritt von fluidem Medium aus einem Innenraum des Behälters kontrolliert wird. Dies kann beispielsweise durch eine optische Detektion des fluiden Mediums außerhalb des Behälters erfolgen, beispielsweise mittels sichtbaren Lichts, ultravioletten Lichts oder infraroten Lichts. Beispielsweise kann zu diesem Zweck eine visuelle Kontrolle durch den Hersteller erfolgen, beispielsweise durch Abfüllpersonal in einer Abfüllanlage, oder es kann eine automatisierte optische Kontrolle erfolgen, beispielsweise mittels einer entsprechenden Bilderkennungsvorrichtung oder ähnlicher Vorrichtungen. Auf diese Weise können Undichtigkeiten frühzeitig erkannt werden, beispielsweise indem ein Aussortieren undichter Transport- und Transferbehältnisse vor einer Lieferung an einen Händler, einen Zwischenhändler oder einen Kunden erfolgt. Auch ein Benutzer hat die Möglichkeit, das Transport- und Transferbehältnis auf Unregelmäßigkeiten hin zu überprüfen, da ein Austritt des fluiden Mediums auch mit bloßem Auge leicht festgestellt werden kann. Somit kann allgemein ein höheres Sicherheits- und Sterilitätslevel erreicht werden.

Das Herstellungsverfahren kann insbesondere, wie oben ausgeführt, derart durchgeführt werden, dass nach Durchführung des Verfahrensschritts d) zumindest der mindestens eine Behälter sowie optional ein oder mehrere weitere Bestandteile des Transport- und Transferbehältnisses in mindestens einer Verpackung aufgenommen werden, insbesondere einem Blisterpack. Diese Verpackung kann beispielsweise für einen Transport des Transport- und Transferbehältnisses zu einem Händler, einem Zwischenhändler oder einem Endkunden eingerichtet sein. Insbesondere kann die Verpackung zumindest teilweise als Sterilverpackung ausgestaltet sein, mit mindestens einem Verpackungsinnenraum, welcher steril gegenüber einer Umgebung abgeschirmt ist. Im Gegensatz zu bekannten Transport- und Transferbehältnissen kann somit eine Aufnahme des Behälters in einem mit dem Überdruck beaufschlagten Zustand, beispielsweise mit gespannter Energieeinheit, in der Verpackung erfolgen, sodass beispielsweise ein Transport des Behälters und/oder eine Lagerung des Behälters in der Verpackung in druckbeaufschlagtem bzw. gespanntem Zustand erfolgen kann.

Wie oben ausgeführt, kann die Energieeinheit insbesondere eine mechanische Energieeinheit sein. Die Energieeinheit kann beispielsweise mechanisch mit dem Behälter gekoppelt werden, beispielsweise indem diese mechanisch auf einen Stopfen des Behälters einwirkt. Die Energieeinheit kann auch ganz oder teilweise bauteilidentisch mit dem Behälter oder Teilen des Behälters sein, beispielsweise indem, wie oben ausgeführt, ein Stopfen des Behälters selbst Bestandteil der Energieeinheit ist, wobei beispielsweise ein mechanischer Energiespeicher, beispielsweise mindestens ein Federelement, mechanisch auf den Stopfen einwirkt.

Der Behälter kann insbesondere mindestens eine Behälterwand mit mindestens einer, vorzugsweise genau einer, Öffnung umfassen, wobei die Öffnung nach Durchführung des Verfahrensschritts c) durch mindestens ein Element mit einem perforierbaren Abschnitt, insbesondere durch mindestens einen perforierbaren Stopfen, verschlossen wird. Dies kann beispielsweise dadurch erfolgen, dass der Behälter, wie oben ausgeführt, als röhrenförmiger, einseitig offener Behälter ausgestaltet ist, wobei die einseitige Öffnung nach Durchführung des Verfahrensschritts c) durch den Stopfen verschlossen werden kann. Der perforierbare Abschnitt, insbesondere der perforierbare Stopfen, kann insbesondere relativ zu der Behälterwand verschiebbar ausgestaltet sein, insbesondere parallel zu einer Achse des Behälters, beispielsweise einer Symmetrieachse oder Rotationsachse des vorzugsweise röhrenförmig ausgestalteten Behälters.

Der Behälter kann insbesondere zumindest teilweise in mindestens einem Außenbehälter aufgenommen werden, insbesondere nach Durchführung des Verfahrensschritts c). So kann das Transport- und Transferbehältnis neben dem Behälter und der Energieeinheit weiterhin mindestens einen Außenbehälter aufweisen. Dieser kann beispielsweise den Behälter vollständig oder teilweise umschließen. Beispielsweise kann der Behälter, wie oben ausgeführt, zumindest teilweise röhrenförmig ausgestaltet sein. Auch der Außenbehälter kann beispielsweise mindestens teilweise röhrenförmig ausgestaltet sein und den Innenbehälter beispielsweise konzentrisch umschließen. Die Energieeinheit kann insbesondere zumindest teilweise in einen Zwischenraum zwischen dem Außenbehälter und dem Behälter angeordnet werden.

Der Verfahrensschritt d), insbesondere die Druckbeaufschlagung, kann vorzugsweise zumindest teilweise während eines Verbindens des Außenbehälters mit dem Behälter und/oder während eines Schließens des Außenbehälters erfolgen, wobei vorzugsweise die Energieeinheit mechanisch gespannt wird. Dies kann beispielsweise dadurch erfolgen, dass bei einem Verbinden des Außenbehälters mit dem Behälter und/oder während eines Schließens des Außenbehälters derart auf die Energieeinheit, beispielsweise auf mindestens einen mechanischen Energiespeicher und besonders bevorzugt mindestens ein Federelement der Energieeinheit, eingewirkt wird, dass diese gespannt wird und/oder gleichzeitig die Druckbeaufschlagung erfolgt.

Die Energieeinheit kann insbesondere eingerichtet sein, um einen Druck auf mindestens einen verschiebbaren Abschnitt des Behälters auszuüben, insbesondere auf einen verschiebbaren perforierbaren Abschnitt und vorzugsweise auf mindestens einen perforierbaren verschiebbaren Stopfen.

Die Energieeinheit kann insbesondere, wie oben ausgeführt, eine mechanische Energieeinheit umfassen oder vollständig als mechanische Energieeinheit ausgestaltet sein, also eine Energieeinheit, welche eingerichtet ist, um auf ausschließlich mechanischem Wege, vorzugsweise ohne elektrische oder elektromechanische Komponenten, die Druckbeaufschlagung und/oder eine Energiespeicherung zu bewirken. Vorzugsweise kann die Energieeinheit als eine manuell bedienbare und manuell spannbare Energieeinheit ausgestaltet sein. Die Energieeinheit kann insbesondere mindestens ein Federelement umfassen, insbesondere mindestens eine Spiralfeder und/oder mindestens eine Schraubendruckfeder.

Umfasst die Energieeinheit mindestens ein Federelement, insbesondere mindestens eine Spiralfeder und/oder mindestens eine Schraubendruckfeder, so kann das Federelement nach Durchführung des Verfahrensschritts d) insbesondere mindestens einen Teil des Behälters gegen mindestens ein Widerlager des Transport- und Transferbehältnisses pressen. Beispielsweise kann das Federelement derart auf den Behälter einwirken, dass mindestens ein verschiebbarer Abschnitt des Behälters, insbesondere ein verschiebbarer perforierbarer Abschnitt und besonders bevorzugt ein verschiebbarer perforierbarer Stopfen, welcher den Behälter verschließt, gegen das Widerlager gepresst wird. Beispielsweise kann, wie oben ausgeführt, der Behälter mindestens ein einseitig geöffneter röhrenförmiger Behälter sein, welcher durch einen verschiebbar gelagerten Stopfen verschlossen wird, wobei der einseitig geschlossene röhrenförmige Behälter beispielsweise mit dem Stopfen gegen das Widerlager gepresst werden. Das Widerlager kann allgemein insbesondere Teil eines des den Behälter zumindest teilweise umschließenden Außenbehälters sein. Beispielsweise kann dieser Außenbehälter, wie oben ausgeführt, ebenfalls zumindest teilweise röhrenförmig ausgestaltet sein, wobei beispielsweise in einem Zwischenraum zwischen dem Außenbehälter und dem Behälter das Federelement vollständig oder teilweise aufgenommen ist. Das Widerlager kann allgemein während eines Entleerungsvorgangs, bei welchem das fluide Medium aus dem Behälter entnommen wird, beispielsweise bei dem oben beschriebenen bestimmungsgemäßen Transfer des fluiden Mediums aus dem Behälter heraus, relativ zu dem Behälter verschiebbar sein. Insbesondere kann das Widerlager während des Entleerungsvorgangs in den Innenraum des Behälters eindringen, beispielsweise indem das Widerlager einen Stempel umfasst, welcher den Stopfen in den Innenraum verschiebt, wobei der Stempel ebenfalls in den Innenraum eindringt.

Das Widerlager, beispielsweise der Stempel, kann insbesondere mindestens eine Perforationsöffnung umfassen, wobei mindestens ein Perforationselement, insbesondere mindestens eine Kanüle, durch die Perforationsöffnung hindurch zu mindestens einem perforierbaren Abschnitt des Behälters, insbesondere zu mindestens einem perforierbaren Stopfen, vordringen kann.

Die Energieeinheit kann insbesondere eingerichtet sein, um während einer Entnahme des fluiden Mediums im Rahmen einer Verwendung des Transport- und Transferbehältnisses ein im Wesentlichen füllstandsunabhängigen Druck auf das fluide Medium auszuüben. So kann die Energieeinheit insbesondere eingerichtet sein, um während der Entnahme des fluiden Mediums zunächst einen Anfangsdruck auf das fluide Medium auszuüben, wobei der Druck auf das fluide Medium während des Entnahmevorgangs um nicht mehr als 50%, vorzugsweise um nicht mehr als 30% und besonders bevorzugt um nicht mehr als 20% oder sogar nicht mehr als 10% von diesem Anfangsdruck abweicht.

In einem weiteren Aspekt der vorliegenden Erfindung wird ein Bereitstellungsverfahren zur Bereitstellung eines fluiden Mediums vorgeschlagen, wobei ein oder mehrere fluide Medien bereitgestellt werden können. Unter einer Bereitstellung ist allgemein ein Vorgang zu verstehen, bei welchem das mindestens eine fluide Medium an einem Einsatzort zur Verfügung gestellt wird, vorzugsweise in einer vorgegebenen Menge und/oder unter Sterilbedingungen. Die Bereitstellung kann beispielsweise nach einem Transport- und/oder einer Lagerung erfolgen. Die Bereitstellung kann beispielsweise an eine Vorrichtung erfolgen. Das Bereitstellungsverfahren umfasst keine therapeutischen und/oder chirurgischen und/oder diagnostischen Verfahrensschritte, die am menschlichen oder tierischen Körper durchgeführt werden. Das Bereitstellungsverfahren selbst kann jedoch Bestandteil eines übergeordneten Verfahrens sein, welches neben dem Bereitstellungsverfahren einen oder mehrere therapeutische und/oder chirurgische und/oder diagnostische Verfahrensschritte, die am menschlichen Körper oder nicht am menschlichen Körper durchgeführt werden, umfassen kann.

Das Bereitstellungsverfahren umfasst folgende Verfahrensschritte, welche vorzugsweise wiederum in der dargestellten Reihenfolge durchgeführt werden, wobei jedoch wiederum auch eine andere Reihenfolge möglich ist, analog zur obigen Beschreibung des Herstellungsverfahrens, und wobei auch ein oder mehrere zusätzliche, nicht genannte Verfahrensschritte durchführbar sind:
- Eine Herstellung eines Transport- und Transferbehältnisses unter Verwendung eines Herstellungsverfahrens gemäß einem der vorhergehenden Ansprüche; und
- Eine Perforation mindestens eines perforierbaren Abschnitts zur Herstellung einer Fluidverbindung zwischen einem Innenraum des Behälters des Transport- und Transferbehältnisses und ein Außenraum zum Zweck einer Entnahme des fluiden Mediums aus dem Behälter.

Ist der Behälter, wie oben ausgeführt, in mindestens einer Verpackung aufgenommen, so kann das Bereitstellungsverfahren vor der Perforation des perforierbaren Abschnitts optional mindestens einen weiteren Verfahrensschritt umfassen, in welchem die Verpackung ganz oder teilweise geöffnet wird und/oder in welchem der Behälter ganz oder teilweise aus der Verpackung entnommen wird. Dieses Öffnen und/oder diese Entnahme können beispielsweise zumindest derart erfolgen, dass der perforierbare Abschnitt zugänglich ist für die Perforation. Beispielsweise kann dieser Schritt ein Öffnen eines Blisterpacks umfassen. Alternativ oder zusätzlich kann bei der Perforation die mindestens eine Verpackung auch ganz oder teilweise mit geöffnet und/oder mit perforiert werden.

Insbesondere kann dieser perforierbare Abschnitt, wie oben ausgeführt, mindestens eine perforierbare Wand des Behälters umfassen, insbesondere mindestens einen perforierbaren Stopfen und vorzugsweise ein verschiebbar in dem Behälter gelagerten perforierbaren Stopfen. Bezüglich möglicher Ausgestaltungen kann auf die obige Beschreibung des Herstellungsverfahrens verwiesen werden. Der perforierbare Abschnitt kann insbesondere elastische Eigenschaften aufweisen, beispielsweise indem ein oder mehrere Elastomermaterialien verwendet werden. Unter einer Perforation ist allgemein ein Vorgang zu verstehen, bei welchem mittels mindestens eines Perforationselements, beispielsweise mittels mindestens eines Nadelements, der perforierbare Abschnitt durchdrungen wird, sodass eine Fluidverbindung hergestellt wird, beispielsweise indem ein hohles Perforationselement verwendet wird, beispielsweise eine Kanüle. Die Perforation erfolgt vorzugsweise reversibel, sodass nach einem Entfernen des Perforationselements aus dem perforierbaren Abschnitt, beispielsweise dem perforierbaren Stopfen, die dabei erzeugte Öffnung wieder verschlossen wird. Dies ermöglicht auch eine portionsweise Entnahme des fluiden Mediums, beispielsweise in konstanten oder auch variablen Portionen.

Unter einer Fluidverbindung ist allgemein eine Verbindung zu verstehen, durch welche fluides Medium aus dem Innenraum des Behälters in einen Außenraum, vorzugsweise einen Außenraum außerhalb des Transport- und Transferbehältnisses, gelangen kann.

Das Bereitstellungsverfahren kann weiterhin einen Transfer zumindest eines Teils des fluiden Mediums durch die Fluidverbindung heraus umfassen, wobei der Transfer vollständig oder teilweise durch die Energieeinheit angetrieben wird. So kann die Energieeinheit beispielsweise das fluide Medium, wie oben ausgeführt, derart mit Druck beaufschlagen, dass das fluide Medium über die Fluidverbindung aus dem Behälter herausgetrieben wird.

Der Transfer kann insbesondere vollständig durch eine im Verfahrensschritt d) in der Energieeinheit gespeicherte Energie, insbesondere eine mechanische Energie, bewirkt werden, sodass vorzugsweise während des Transfers keine zusätzliche, nicht in der Energieeinheit gespeicherte Energie in das Transport- und Transferbehältnis eingebracht werden muss.

Die Bereitstellung kann insbesondere zumindest teilweise in eine Injektionskanüle hinein erfolgen. Mittels dieser Injektionskanüle kann das fluide Medium beispielsweise an eine weitere Vorrichtung bereitgestellt werden oder auch im Rahmen eines übergeordneten Verfahrens verwendet werden, beispielsweise eines Injektionsverfahrens. So kann in einer bevorzugten Ausgestaltung des Bereitstellungsverfahrens die Bereitstellung zumindest teilweise in mindestens ein von dem Transport- und Transferbehältnis getrennt ausgebildetes Behältnis hinein erfolgen. Beispielsweise kann es sich hierbei um eine Flasche, ein Schraubdeckelgläschen, ein Schnappdeckelgläschen oder eine andere Art von Behältnis handeln.

In einer weiteren bevorzugten Ausgestaltung des Bereitstellungsverfahrens wird das Bereitstellungsverfahren wiederholt durchgeführt, wobei nacheinander eine Perforation des perforierbaren Abschnitts mehrerer Behälter zur Herstellung einer Fluidverbindung zwischen einem Innenraum der Behälter und einem Außenraum zum Zweck einer Entnahme des fluiden Mediums aus den Behältern erfolgt, wobei ein Wechsel der Behälter ohne Eintrag von Gasblasen erfolgt. Auf diese Weise kann beispielsweise ein erstes Transport- und Transferbehältnis hergestellt werden, anschließend kann eine Perforation mindestens eines perforierbaren Abschnitts zur Herstellung einer Fluidverbindung zwischen einem Innenraum des Behälters des ersten Transport- und Transferbehältnisses und einem Außenraum zum Zweck einer Entnahme des fluiden Mediums aus dem ersten Behälter erfolgen. Gleichzeitig, zeitlich überlappend oder anschließend kann eine Herstellung mindestens eines weiteren Transport- und Transferbehältnisses erfolgen, gefolgt von einer Perforation mindestens eines perforierbaren Abschnitts zur Herstellung einer Fluidverbindung zwischen einem Innenraum des Behälters des weiteren Transport- und Transferbehältnisses und einem Außenraum, zum Zweck einer Entnahme des fluiden Mediums aus dem weiteren Behälter. So kann beispielsweise ein Behälterwechsel ohne Eintrag von Gasblasen in ein System, welches das Transport- und Transferbehältnis verwendet, erfolgen.

Das Bereitstellungsverfahren kann insbesondere derart erfolgen, dass die Bereitstellung zumindest teilweise in mindestens eine flexible Verbindung, insbesondere eine flexible Fluidverbindung und besonders bevorzugt in mindestens eine Schlauchverbindung, hinein erfolgt. So kann beispielsweise das Bereitstellungsverfahren in eine Schlauchverbindung eines Infusionskits hinein erfolgen. So kann die flexible Verbindung, in welche hinein das Bereitstellungverfahren erfolgt, in mindestens einem Infusionskit münden, wobei das Infusionskit mindestens eine Infusionskanüle und mindestens ein Pflaster umfassen kann. Beispielsweise kann mit dieser Infusionskanüle in einem übergeordneten Infusionsverfahren, welches das Bereitstellungsverfahren umfasst, eine Injektion des fluiden Mediums in ein Körpergewebe eines Benutzers hinein erfolgen.

Das Bereitstellungsverfahren kann weiterhin derart durchgeführt werden, dass dieses mindestens einen Unterbrechungsschritt umfasst. In diesem Unterbrechungsschritt kann die Perforation des perforierbaren Abschnitts aufgehoben werden, und die Fluidverbindung kann dadurch unterbrochen werden. Beispielsweise kann dies dadurch erfolgen, dass der perforierbare Abschnitt, wie oben ausgeführt, flexible Eigenschaften aufweist. Beispielswese kann ein Perforationselement aus dem preforierbaren Abschnitt herausgezogen werden oder auf andere Weise entfernt werden, wobei eine verbleibende Öffnung aufgrund der flexiblen Eigenschaften des perforierbaren Elements geschlossen und die Fluidverbindung unterbrochen wird. Beispielsweise kann diese durch Verwendung eines perforierbaren flexiblen Stopfens erfolgen. Ein Vorteil des Transport- und Transferbehältnisses sowie des beschriebenen Bereitstellungsverfahren kann insbesondere, wie oben ausgeführt, darin bestehen, dass die Druckbeaufschlagung des fluiden Mediums in dem Behälter unabhängig von der Herstellung der Fluidverbindung erfolgen kann, insbesondere mechanisch entkoppelt. So kann beispielsweise die Druckbeaufschlagung andauern, wohingegen die Herstellung der Fluidverbindung beispielsweise ausschließlich durch die Perforation hergestellt wird, welche mechanisch von der Druckbeaufschlagung entkoppelt sein kann und welche beispielsweise reversibel ist. So kann, beispielsweise unter Aufrechterhaltung eines Überdrucks in dem Behälter, die Fluidverbindung hergestellt und optional wieder unterbrochen werden, um anschließend optional ein oder mehrere weitere Male wieder hergestellt zu werden, beispielsweise durch eine erneute Perforation.

Wie oben ausgeführt, kann das Bereitstellungsverfahren insbesondere derart ausgestaltet werden, dass die Bereitstellung keinen an einem menschlichen oder tierischen Körper durchgeführten therapeutischen, diagnostischen oder chirurgischen Verfahrensschritt umfasst. Das Bereitstellungsverfahren kann jedoch Bestandteil eines übergeordneten Injektionsverfahrens sein, welches mindestens ein Bereitstellungsverfahren gemäß einer oder mehreren der oben beschriebenen Ausgestaltungen umfassen kann. Weiterhin kann das Injektionsverfahren mindestens einen Injektionsschritt umfassen, wobei das mittels des Bereitstellungsverfahrens bereitgestellte fluide Medium in ein Körpergewebe eines menschlichen oder tierischen Körpers injiziert wird.

Das Bereitstellungsverfahren umfasst, wie oben ausgeführt, mindestens eine Perforation mindestens eines perforierbaren Abschnitts zur Herstellung einer Fluidverbindung zwischen dem Innenraum des Behälters und dem Außenraum, zum Zweck einer Entnahme des fluiden Mediums aus dem Behälter. So kann in dem Bereitstellungsverfahren insbesondere mindestens ein Perforationselement zur Herstellung der Fluidverbindung verwendet werden, insbesondere mindestens eine Kanüle. Das Perforationselement kann beispielsweise an einem dem Behälter zuweisenden Ende eine Spitze aufweisen, mittels derer die Perforation des perforierbaren Abschnitts, beispielsweise des perforierbaren Stopfens, erfolgen kann. Optional kann das Perforationselement jedoch auch an einem von dem Behälter abgewandten Ende eine Spitze umfassen, insbesondere eine Injektionsnadel und besonders bevorzugt eine Injektionskanüle. Die Injektionsnadel kann zur Injektion des fluiden Mediums in das Körpergewebe verwendet werden. Insbesondere kann das Perforationselement eine Injektionsnadel umfassen, vorzugsweise eine Injektionskanüle, mit zwei an einander gegenüberliegenden Enden angeordneten Spitzen, beispielsweise einer Spitze, welche dem Behälter zuweist, und einer Spitze, welche dem Körpergewebe, in welches hinein die Injektion erfolgen soll, zuweist.

Das Perforationselement kann somit, wie oben ausgeführt, selbst als Injektionsnadel ausgestaltet sein und/oder als Injektionsnadel verwendet werden. Alternativ oder zusätzlich kann das Perforationselement jedoch auch über mindestens eine flexible Verbindung, insbesondere über mindestens eine Schlauchverbindung und vorzugsweise über mindestens eine sterile Schlauchverbindung, mit mindestens einem Injektionselement, insbesondere einer Injektionskanüle, verbunden werden. So kann beispielsweise die Perforation von der eigentlichen Injektion in das Körpergewebe räumlich getrennt ausgestaltet werden, beispielsweise mit einem Abstand von mindestens 5 cm, vorzugsweise mindestens 10 cm, besonders bevorzugt mindestens 20 cm und insbesondere mindestens 50 cm Abstand. So können für die Perforation und für die Injektion getrennte Elemente verwendet werden, beispielsweise getrennte Kanülen, welche über die mindestens eine flexible Verbindung miteinander verbunden sein können.

Das Injektionsverfahren kann insbesondere derart ausgestaltet werden, dass ein Transfer des fluiden Mediums aus dem Behälter heraus und der Injektionsschritt räumlich voneinander getrennt durchgeführt werden. Insbesondere kann eine räumliche Trennung von mindestens 30 mm, vorzugsweise von mindestens 50 mm und besonders bevorzugt von mindestens 100 mm auftreten, beispielsweise die oben beschriebene räumliche Trennung mit den oben genannten bevorzugten Abständen zwischen der Perforation und der Injektion.

Weiterhin können die Injektion und die Perforation auch zeitlich voneinander getrennt ausgestaltet werden, beispielsweise im Gegensatz zu üblichen Injektionsverfahren mittels Spritzen oder Autoinjektoren. So kann beispielsweise ein Einstich mindestens eines Injektionselements in das Körpergewebe erfolgen und anschließend zeitversetzt der Transfer des fluiden Mediums aus dem Behälter heraus in das Körpergewebe, beispielsweise mit einem Zeitversatz von mindestens 5 Sekunden, insbesondere von mindestens 10 Sekunden. Auch eine andere Ausgestaltung einer zeitlichen Trennung zwischen einem Transfer des fluiden Mediums aus dem Behälter heraus und einem Einstich in das Körpergewebe ist grundsätzlich möglich.

Das Injektionsverfahren kann weiterhin derart durchgeführt werden, dass ein Einstich mindestens eines Injektionselements in das Körpergewebe und den Transfer des fluiden Mediums aus dem Behälter heraus separate Arbeitsschritte sind, vorzugsweise mechanisch voneinander entkoppelte separate Arbeitsschritte. Diese Entkopplung kann beispielsweise, wie oben ausgeführt, über die mindestens eine optionale flexible Verbindung, insbesondere über die mindestens eine optionale Schlauchverbindung und besonders bevorzugt über die mindestens eine sterile Schlauchverbindung, erfolgen.

In einem weiteren Aspekt der vorliegenden Erfindung wird ein Transport- und Transferbehältnis zur Lagerung und Bereitstellung mindestens eines fluiden Mediums vorgeschlagen. Insbesondere kann das Transport- und Transferbehältnis zur Lagerung und Bereitstellung mindestens einer sterilen Flüssigkeit, insbesondere eines Diagnostikums und/oder Therapeutikums, eingerichtet sein. Für eine Definition des Begriffs des Transport- und Transferbehältnisses sowie bezüglich möglicher Ausgestaltungen desselben kann auf die obige Beschreibung des Herstellungsverfahrens verwiesen werden. Auch andere Ausgestaltungen sind jedoch grundsätzlich möglich. Das Transport- und Transferbehältnis kann insbesondere nach einem Herstellungsverfahren gemäß einer oder mehreren der oben beschriebenen Ausgestaltungen oder gemäß einem oder mehrere der nachfolgend noch beschriebenen Beispiele herstellbar sein.

Das Transport- und Transferbehältnis umfasst:
A) mindestens einen Behälter mit dem darin aufgenommenen fluiden Medium, wobei der Behälter eine Behälterwand und mindestens einen perforierbaren Abschnitt aufweist, insbesondere mindestens einen perforierbaren Stopfen, wobei mittels einer Perforation des perforierbaren Abschnitts eine Fluidverbindung zu einem Innenraum des Behälters herstellbar ist zum Zweck einer Entnahme des fluiden Mediums aus dem Behälter;
B) mindestens eine integrierte Energieeinheit, wobei die Energieeinheit eingerichtet ist, um bei einer Verwendung des Transport- und Transferbehältnisses eine - vorzugsweise vollständige - Entleerung des fluiden Mediums aus dem Behälter zu bewirken, wobei die Energieeinheit eingerichtet ist, um das fluide Medium in dem Behälter mit einem Überdruck zu beaufschlagen, wobei der Überdruck bis zu einer Entnahme des fluiden Mediums aufrechtgehalten werden kann und/oder aufrechtgehalten wird,
wobei das Transport- und Transferbehältnis weiterhin mindestens eine Verpackung aufweist, wobei zumindest der Behälter in dem mit dem Überdruck beaufschlagten Zustand in der Verpackung aufgenommen ist.

Das Transport- und Transferbehältnis kann insbesondere derart ausgestaltet sein, dass das fluide Medium während eines Transports und einer Lagerung ausschließlich mit einer Behälterwand des Behälters und einem Verschlusselement des Behälters in Berührung kommt.

Bezüglich möglicher Ausgestaltungen der Verpackung kann auf die obige Beschreibung der optionalen Ausgestaltungen des Herstellungsverfahrens verwiesen werden.

Die Energieeinheit kann insbesondere eingerichtet sein, um auf den perforierbaren Abschnitt mechanisch einzuwirken. Der perforierbare Abschnitt kann insbesondere ganz oder teilweise identisch mit einem Verschlusselement sein, beispielsweise einem Stopfen und insbesondere einem perforierbaren, verschiebbar in dem Behälter aufgenommenen Stopfen. Somit kann die Energieeinheit auch eingerichtet sein, um auf das Verschlusselement mechanisch einzuwirken. Unter einer mechanischen Einwirkung ist allgemein eine Beaufschlagung mit einer Kraft zu verstehen, vorzugsweise derart, dass der perforierbare Abschnitt und insbesondere das Verschlusselement bewegt werden. Insbesondere können der perforierbare Abschnitt und/oder das Verschlusselement einen oder mehrere Stopfen umfassen, beispielsweise einen oder mehrere perforierbare Stopfen. So kann die Energieeinheit eingerichtet sein, um auf den Stopfen, insbesondere den perforierbaren Stopfen, mechanisch einzuwirken.

Unter einer bevorzugten Ausgestaltung, bei welcher das fluide Medium ausschließlich mit der Behälterwand und dem Verschlusselement des Behälters in Berührung kommt, ist eine Ausgestaltung zu verstehen, bei welcher das fluide Medium vor einer Verwendung desselben maximal mit den Elementen und/oder Oberflächen in Berührung kommt, welche den Innenraum des Behälters definieren und begrenzen. Beispielsweise steht das fluide Medium dementsprechend nicht mit Ventilen, Schläuchen oder anderen fluidischen Elementen in Kontakt.

Bezüglich möglicher Ausgestaltungen des Behälters kann auf die obige Beschreibung verwiesen werden. Unter einer Behälterwand kann allgemein eine dem Innenraum zuweisende Innenseite des Behälters zu verstehen sein, welche beispielsweise mindestens einen zylindrischen Abschnitt und/oder mindestens einen ebenen Abschnitt aufweisen kann, insbesondere wenn der Behälter röhrenförmig ausgestaltet ist. Unter einem Verschlusselement kann allgemein ein Element verstanden werden, welches eingerichtet ist, um den Behälter, wie oben beschrieben, zu verschließen. Beispielsweise kann dieses Verschlusselement einen Stopfen umfassen und/oder ein Stopfen sein und/oder kann ein perforierbares Verschlusselement umfassen, insbesondere einen perforierbaren Stopfen. So kann das fluide Medium beispielsweise ausschließlich mit der Behälterwand und dem Stopfen in Berührung kommen, bevor dieses aus dem Innenraum entnommen wird.

Der Behälter sowie optional ein oder mehrere weitere Bestandteile des Transport- und Transferbehältnisses sind ganz oder teilweise in mindestens einer Verpackung gemäß der obigen Definition aufgenommen sein.

Die Verpackung ist im Sinne der obigen Definition Bestandteil des Transport- und Transferbehältnisses, auch wenn diese beispielsweise vor Benutzung des Transport- und Transferbehältnisses und/oder vor einer Entnahme des fluiden Mediums ganz oder teilweise entfernt werden kann. So können die übrigen Bestandteile des Transport- und Transferbehältnisses mit Ausnahme der Verpackung mindestens eine Nutzungseinheit bilden, welche, beispielsweise nach Öffnung und/oder vollständiger oder teilweiser Entfernung der Verpackung, für die Entnahme des fluiden Mediums genutzt werden kann. Beispielsweise können sämtliche Komponenten des Transport- und Transferbehältnisses, mit Ausnahme der Verpackung, in ein und derselben Verpackung aufgenommen sein. Insbesondere kann auch ein Außenbehälter, welcher den Behälter sowie optional die Energieeinheit ganz oder teilweise umschließen kann, in der Verpackung aufgenommen sein. Insbesondere kann die Verpackung eine luftdichte und/oder keimdichte .Verpackung sein oder umfassen. Bezüglich möglicher Ausgestaltungen der Verpackung kann auf die obige Beschreibung verwiesen werden. Unter einer zumindest teilweisen Aufnahme des Behälters sowie optional einer oder mehrerer weiterer Komponenten des Transport- und Transferbehältnisses in der Verpackung kann im Rahmen der vorliegenden Erfindung allgemein beispielsweise eine Ausgestaltung zu verstehen sein, bei welcher zumindest ein Bereich der genannten, in der Verpackung aufgenommenen Komponente und vorzugsweise die gesamte Komponente von der Verpackung umhüllt ist. Beispielsweise können zumindest der Behälter und optional weiterhin die Energieeinheit jeweils vollständig oder teilweise von derselben Verpackung umhüllt sein, wohingegen weitere Komponenten optional auch separat bereitgestellt und/oder verpackt sein können. Insbesondere können der Behälter und die Energieeinheit gemeinsam in einer Verpackung aufgenommen sein. Beispielsweise können der Behälter sowie optional die Energieeinheit in einem Zustand in der Verpackung aufgenommen sein, in welchem das fluide Medium in dem Behälter durch die Energieeinheit mit dem Überdruck beaufschlagt ist. Somit kann beispielsweise ein mechanischer Energiespeicher der Energieeinheit, beispielsweise mindestens ein Federelement, in einem gespannten Zustand in der Verpackung aufgenommen sein.

Das Transport- und Transferbehältnis kann insbesondere derart ausgestaltet sein, dass das fluide Medium blasenfrei in diesem aufgenommen ist. Unter einer blasenfreien Aufnahme wird eine Ausgestaltung verstanden, bei welcher das fluide Medium keine Gasblasen enthält und nicht mit Gasblasen in Kontakt steht, solange das fluide Medium in dem Behälter aufgenommen ist. Unter Gasblasen können dabei allgemein zusammenhängende Gasvolumina mit einem Volumen von mindestens 5 x 10⁻⁷ mm³ verstanden werden.

Insbesondere kann der Behälter derart ausgestaltet sein, dass dieser keine Fluidverbindung zwischen dem Innenraum des Behälters und einem Außenraum aufweist, mit Ausnahme der durch die Perforation des perforierbaren Abschnitts herstellbaren Fluidverbindung, beispielsweise ausschließlich zur Entnahme des fluiden Mediums. Insbesondere kann der Behälter derart ausgestaltet sein, dass keine Ventile oder ähnliche Fluidverbindungen vorgesehen sind. Für mögliche Ausgestaltungen des Behälters und des perforierbaren Abschnitts, welcher insbesondere Bestandteil eines perforierbaren Stopfens sein kann, kann auf die obige Beschreibung verwiesen werden. Ebenso kann bezüglich möglicher Ausgestaltungen der Energieeinheit auf die obige Beschreibung verwiesen werden.

Unter einer vollständigen Entleerung des fluiden Mediums ist eine Entleerung des Behälters zu verstehen, welche dergestalt ist, dass bei einer Herstellung einer Fluidverbindung zwischen einem Außenraum und dem Innenraum des Behälters, insbesondere bei einer Perforation des perforierbaren Abschnitts, und bei einer Aufrechterhaltung dieser Fluidverbindung allein aufgrund der Energieeinheit und beispielsweise der in dieser Energieeinheit gespeicherten mechanischen Energie eine Entleerung des Behälters auftritt, bei welcher mindestens 90% des fluiden Mediums, welches zuvor in dem Behälter enthalten war, aus dem Behälter entfernt wird, vorzugsweise mindestens 95% und besonders bevorzugt mindestens 98%. Beispielsweise können Restmengen in dem Behälter dabei toleriert werden, welche vorzugsweise 10% des ursprünglichen Fluidvolumens nicht überschreiten, insbesondere 5% nicht überschreiten und besonders bevorzugt 2% oder sogar 1% nicht überschreiten.

Die Energieeinheit ist vorzugsweise eingerichtet, um auf den verschiebbaren Abschnitt mechanisch einzuwirken beispielsweise auf einen perforierbaren oder nicht-perforierbaren verschiebbaren Abschnitt. Beispielsweise kann dies dadurch erfolgen, dass die Energieeinheit, wie oben beschrieben, den verschiebbaren Abschnitt mit einer Kraft beaufschlagt, die mindestens eine Kraftkomponente hin zu dem Innenraum des Behälters aufweist. Beispielsweise kann der verschiebbare Abschnitt zu dem Innenraum des Behälters hin geschoben werden. Dies kann, wie oben ausgeführt, beispielsweise dadurch erfolgen, dass die Energieeinheit mindestens ein Federelement umfasst, beispielsweise eine Spiralfeder und/oder mindestens eine Schraubendruckfeder, welche direkt oder indirekt auf den verschiebbaren Abschnitt einwirkt, derart, dass dieser mit der genannten Kraft beaufschlagt wird. Dies kann beispielsweise dadurch erfolgen, dass das Federelement direkt auf den verschiebbaren Abschnitt einwirkt oder dass das Federelement derart auf den Behälter einwirkt, dass der verschiebbare Abschnitt beispielsweise gegen ein Widerlager gepresst wird. Der verschiebbare Abschnitt kann insbesondere, wie oben beschrieben, mindestens einen verschiebbaren Stopfen umfassen, beispielsweise einen perforierbaren verschiebbaren Stopfen, welcher vorzugsweise beweglich in dem Behälter gelagert ist. Für mögliche Ausgestaltungen kann auf die obige Beschreibung verwiesen werden. Die Energieeinheit kann insbesondere derart eingerichtet sein, dass die mechanische Einwirkung nach Herstellung des Transport- und Transferbehältnisses bis zu einer bestimmungsgemäßen Verwendung desselben und einer vollständigen oder teilweisen Entnahme des fluiden Mediums aus dem Behälter dauerhaft besteht.

Das Transport- und Transferbehältnis kann, wie oben ausgeführt, insbesondere herstellbar sein nach einem Herstellungsverfahren gemäß einem oder mehreren der oben oder nachfolgend noch beschriebenen Ausgestaltungen. Der perforierbare Abschnitt kann somit insbesondere mindestens einen, vorzugsweise genau einen, zu einer Behälterwand des Behälters verschiebbar gelagerten Stopfen umfassen oder kann vorzugsweise genau ein relativ zu der Behälterwand verschiebbar gelagerter Stopfen sein. Beispielsweise kann die Behälterwand, wie oben ausgeführt, im Wesentlichen röhrenförmig ausgestaltet sein, beispielsweise mit einer zylindrischen Innenwand. So kann der Behälter beispielsweise eine Form einer einseitig geschlossenen Röhre aufweisen, wobei eine dem geschlossenen Ende gegenüber liegende Öffnung beispielsweise durch den Stopfen verschlossen ist. Allgemein kann der Behälter somit insbesondere ein röhrenförmiger, einseitig mit einer Öffnung versehener Behälter sein.

Das fluide Medium kann insbesondere nach Aufnahme in dem Behälter ausschließlich mit der Behälterwand und dem perforierbaren Abschnitt in Kontakt stehen. So kann beispielsweise das Transport- und Transferbehältnis derart ausgestaltet sein, dass das fluide Medium ausschließlich mit den beiden genannten Elementen in Kontakt steht, also beispielsweise ausschließlich mit der Behälterwand und dem Stopfen, nicht hingegen mit weiteren Elementen wie beispielsweise Ventilen, Schlauchverbindungen oder ähnlichen Elementen, bis hin zu dem Zeitpunkt, zu welchem das fluide Medium aus dem Behälter heraustransferiert wird.

Das Transport- und Transferbehältnis kann insbesondere, wie oben ausgeführt, derart ausgestaltet sein, dass dieses und insbesondere der Behälter keine Ventile aufweist. Das Transport- und Transferbehältnis kann insbesondere derart ausgestaltet sein, dass der Behälter vor einer Perforation des perforierbaren Abschnitts ohne Fluidverbindung zwischen einem das fluide Medium aufnehmenden Innenraum und des Behälters und einer Umgebung des Behälters ist. Insbesondere kann das Transport- und Transferbehältnis derart ausgestaltet sein, dass die Fluidverbindung ausschließlich durch eine Perforation des perforierbaren Abschnitts herstellbar ist, zumindest bei einer zerstörungsfreien, stimmungsgemäßen Verwendung des Transport- und Transferbehältnisses.

Weitere mögliche Ausgestaltungen betreffen die oben beschriebene Energieeinheit. So kann die Energieeinheit insbesondere eingerichtet sein, um den Überdruck nach einer Herstellung des Transport- und Transferbehältnisses während einer Lagerung des Transport- und Transferbehältnisses für eine Zeitdauer von mindestens zwei Wochen, vorzugsweise von mindestens drei Wochen, mindestens vier Wochen, mindestens einen Monat, mindestens zwei Monate, mindestens ein Jahr oder sogar mindestens mehrere Jahre aufrechtzuerhalten, insbesondere auf einem Druckniveau von mindestens 50%, vorzugsweise von mindestens 70% und besonders bevorzugt von mindestens 80% eines Anfangsdrucks. Wie oben ausgeführt, kann dies insbesondere durch eine entsprechende Ausgestaltung eines Federelements erfolgen, wobei das Federelement beispielsweise mit einer geringen Hysterese ausgestaltet wird und einer entsprechenden dauerhaften Steifigkeit, welche beispielsweise durch eine entsprechende Materialauswahl oder eine entsprechende Geometrie des Federelements herstellbar ist. Weiterhin kann die Aufrechterhaltung des Druckniveaus auch beispielsweise durch eine entsprechende Dichtigkeit des Behälters unterstützt werden.

Der Behälter des Transport- und Transferbehältnisses, wobei ein oder mehrere derartiger Behälter vorgesehen sein können, kann insbesondere vollständig oder teilweise mit dem fluiden Medium befüllt sein. Dies kann beispielsweise, wie oben ausgeführt, derart erfolgen, dass eine ursprünglich vorhandene Gasblase in dem fluiden Medium sich nach einer Verweildauer vollständig aufgelöst hat, beispielsweise nach einer Verweildauer von ein bis drei Tagen. Diese Auflösung kann beispielsweise durch eine entsprechende Gaslöslichkeit erfolgen, welche üblicherweise eine Funktion der Temperatur und des Drucks ist, insbesondere des Drucks, mittels dessen durch die Energieeinheit das fluide Medium in dem Behälter beaufschlagt ist.

Die Energieeinheit kann, wie oben ausgeführt, insbesondere mindestens ein Federelement umfassen, wobei das Federelement direkt oder indirekt auf den perforierbaren Abschnitt einwirkt. Unter einem Einwirken kann im Rahmen der vorliegenden Erfindung insbesondere eine Beaufschlagung des perforierbaren Abschnitts mit einer Kraft verstanden werden, insbesondere eine Beaufschlagung mit einer Kraft mit mindestens einer Kraftkomponente hin zu dem Innenraum des Behälters. Diese Kraftbeaufschlagung kann direkt oder indirekt erfolgen, beispielsweise indem das Federelement direkt auf den perforierbaren Abschnitt drückt und/oder indem das Federelement den perforierbaren Abschnitt gegen mindestens ein Widerlager drückt. Dieses Drücken kann wiederum direkt oder indirekt erfolgen, beispielsweise indem das Federelement auf den Behälter einwirkt, beispielsweise eine Behälterwand, wobei über die Behälterwand wiederum der perforierbare Abschnitt gegen das Widerlager gedrückt wird.

Der Behälter und vorzugsweise das gesamte Transport- und Transferbehältnis können insbesondere frei von elektrischen Komponenten sein. So kann beispielsweise das Transport- und Transferbehältnis, wie oben ausgeführt, vollständig als mechanische Einheit ausgestaltet sein.

Das Transport- und Transferbehältnis kann weiterhin mindestens einen Außenbehälter umfassen, wie oben ausgeführt, welcher beispielsweise ebenfalls ganz oder teilweise in der mindestens einen Verpackung aufgenommen sein kann. So kann der Außenbehälter beispielsweise derart ausgestaltet sein, dass der Behälter zumindest teilweise in dem Außenbehälter aufgenommen ist. Die Energieeinheit kann, wie oben ausgeführt, insbesondere zumindest teilweise in eine, Zwischenraum zwischen dem Außenbehälter und dem Behälter angeordnet sein, beispielsweise in mindestens einem Ringspalt zwischen dem Außenbehälter und dem Behälter. Dies kann beispielsweise dadurch erfolgen, dass die Energieeinheit mindestens ein Federelement, insbesondere mindestens eine Spiralfeder und/oder mindestens eine Schraubendruckfeder, umfasst, wobei das Federelement beispielsweise in dem Ringspalt aufgenommen ist. Der Außenbehälter kann den Behälter insbesondere zumindest teilweise röhrenförmig umschließen. Der Außenbehälter und die Energieeinheit können insbesondere derart eingerichtet sein, dass während eines Verbindens des Außenbehälters mit dem Behälter und/oder während eines Schließens des Außenbehälters die Druckbeaufschlagung des fluiden Mediums erfolgt, wobei vorzugsweise die Energieeinheit mechanisch gespannt wird. Dieses Schließen und/oder Verbinden kann beispielsweise mittels eines oder mehrerer Verbindungselemente erfolgen, beispielsweise durch Herstellung mindestens einer formschlüssigen und/oder kraftschlüssigen und/oder stoffschlüssigen Verbindung. Insbesondere können hierfür Schraubverbindungen, Rastverbindungen, oder ähnliche Verbindungen verwendet werden. Zu diesem Zweck können der Außenbehälter und/oder der Behälter eine oder mehrere Verbindungselemente aufweisen. Der Außenbehälter kann insbesondere mindestens zwei miteinander verbindbare Behälterteile aufweisen, wobei das Transport- und Transferbehältnis derart eingerichtet ist, dass bei einem Verbinden der mindestens zwei Behälterteile die Energieeinheit mechanisch gespannt wird. Dies kann beispielsweise dadurch erfolgen, dass die Energieeinheit mindestens ein Federelement aufweist, welches in dem Außenbehälter aufgenommen wird, beispielsweise in einem Zwischenraum zwischen dem Außenbehälter und dem Behälter. Bei einem Verbinden der Behälterteile kann ein Innenraum des Außenbehälters in seinem Volumen verkleinert werden, sodass das Federelement beispielsweise zusammengepresst wird und dadurch mechanisch gespannt wird. Das Verbinden der mindestens zwei Behälterteile kann insbesondere über eine Verbindung erfolgen, ausgewählt aus einer Schraubverbindung und einer Rastverbindung. Auch andere Verbindungen sind jedoch grundsätzlich möglich.

Die Energieeinheit kann insbesondere eine rein mechanische Energieeinheit sein, ohne elektrische und/oder elektromechanische Komponenten, vorzugsweise eine manuell bedienbare Energieeinheit, welche jedoch vorzugsweise eine manuell bedienbare Energieeinheit, welche jedoch vorzugsweise automatisch spannbar ist, beispielsweise automatisch bei einem Schließen des Außenbehälters und/oder bei einem Verbinden des Außenbehälters mit dem Behälter. Unter einer rein mechanischen Energieeinheit ist eine Energieeinheit zu verstehen, bei welcher die oben beschriebene Druckbeaufschlagung und/oder die oben beschriebene Energiespeicherung auf rein mechanischem Wege erfolgt, beispielsweise über ein oder mehrere rein mechanische Federelemente zur Druckbeaufschlagung und/oder zur Energiespeicherung.

So kann die Energieeinheit insbesondere, wie oben ebenfalls ausgeführt, mindestens ein Federelement umfassen, insbesondere mindestens eine Spiralfeder und/oder mindestens eine Schraubendruckfeder. Das Federelement kann vorzugsweise aus einem metallischen Material hergestellt sein, vorzugsweise aus Federstahl. Grundsätzlich ist jedoch, alternativ oder zusätzlich, auch die Verwendung anderer Arten von Materialien möglich, vorzugsweise Materialien mit hoher Elastizität, beispielsweise die Verwendung eines oder mehrerer Elastomermaterialien. Das Federelement kann insbesondere eingerichtet sein, um mindestens einen Teil des Behälters, insbesondere den perforierbaren Abschnitt, gegen mindestens ein Widerlager des Transport- und Transferbehältnisses zu pressen. Beispielsweise kann dieses Widerlager Bestandteil eines Außenbehälters sein, welcher den Behälter vollständig oder teilweise umschließt. Das Federelement kann insbesondere derart auf den Behälter einwirken, dass mindestens ein verschiebbarer perforierbarer Stopfen gegen das Widerlager gepresst wird. Zu diesem Zweck kann das Federelement beispielsweise das Widerlager gegen den perforierbaren Stopfen pressen und/oder den Behälter derart mit einer Kraft beaufschlagen, dass der perforierbare Stopfen gegen das Widerlager gepresst wird. Auch Kombinationen der genannten Möglichkeiten sind denkbar.

Das Widerlager kann insbesondere, wie oben ausgeführt, Bestandteil eines den Behälter zumindest teilweise umschließenden Außenbehälters sein. Das Widerlager kann insbesondere während eines Entleerungsvorgangs, bei welchem das fluide Medium aus dem Behälter entnommen wird, relativ zu dem Behälter verschiebbar sein und kann vorzugsweise in einen Innenraum des Behälters eindringen. So kann das Widerlager beispielsweise einen Stempel umfassen, welcher von seinem Außendurchmesser her kleiner dimensioniert ist als ein Innendurchmesser des Behälters und welcher auf den perforierbaren Stopfen einwirken kann. Das Widerlager kann insbesondere mindestens eine Perforationsöffnung umfassen, wobei mindestens ein Perforationselement, insbesondere mindestens eine Kanüle, durch die Perforationsöffnung zu dem mindestens einen perforierbaren Abschnitt des Behälters vordringen kann, insbesondere zu dem mindestens einen perforierbaren Stopfen. So kann das Widerlager beispielsweise als Stempel ausgestaltet sein, wobei die Perforationsöffnung beispielsweise einen Kanal in dem Stempel aufweist, beispielsweise einen zentralen Kanal, vorzugsweise einen Kanal, welcher parallel zu einer Achse des Behälters, beispielsweise einer Rotationsachse, ausgerichtet ist. Allgemein kann bei dieser oder auch bei anderen Ausgestaltungen der vorliegenden Erfindung der Behälter relativ zu dem optionalen Außenbehälter gelagert sein. So kann sich beispielsweise während des Entleerungsvorgangs der Behälter relativ zu dem Außenbehälter verschieben.

Das Perforationselement kann insbesondere Bestandteil des Transport- und Transferbehältnisses sein. So kann das Transport- und Transferbehältnis allgemein mindestens ein Perforationselement zur Perforation des mindestens einen perforierbaren Abschnitts umfassen, beispielsweise ein Perforationselement, welches in einem Außenbehälter des Transport- und Transferbehältnisses relativ zu dem Behälter und/oder relativ zu dem optionalen Außenbehälter beweglich gelagert ist. Das Perforationselement kann beispielsweise manuell oder auf andere Weise angetrieben werden, um die Perforation durchzuführen. Allgemein kann das Transport- und Transferbehältnis somit mindestens ein Perforationselement umfassen, welches beweglich, insbesondere verschiebbar, in dem Transport- und Transferbehältnis gelagert ist, insbesondere verschiebbar in einem Außenbehälter, welcher den Behälter vollständig oder teilweise umschließt.

Die Energieeinheit kann insbesondere eingerichtet sein, um während einer Entnahme des fluiden Mediums im Rahmen einer Verwendung des Transport- und Transferbehältnisses einen im Wesentlichen Füllstands-unabhängigen Druck auf das fluide Medium auszuüben.

Bezüglich möglicher Ausgestaltungen dieses Aspekts kann auf die obige Beschreibung verwiesen werden.

Das Transport- und Transferbehältnis kann allgemein, wie oben beschrieben, insbesondere mindestens ein Perforationselement zur Perforation des perforierbaren Abschnitts aufweisen, insbesondere mindestens eine Kanüle. Auch dieses Perforationselement kann ganz oder teilweise in der Verpackung aufgenommen sein. Beispielsweise kann dieses Perforationselement vollständig oder teilweise in einem Innenraum eines Außenbehälters gelagert sein, insbesondere verschiebbar, wobei der Außenbehälter beispielsweise den Behälter vollständig oder teilweise umschließt.

Das Transport- und Transferbehältnis kann weiterhin mindestens ein Injektionselement umfassen, insbesondere mindestens eine Injektionskanüle, wobei das Injektionselement eingerichtet ist, um in ein menschliches oder tierisches Körpergewebe und/oder in ein von dem Behälter getrennt ausgebildetes Gefäß, insbesondere ein Behältnis und/oder eine andere Art von Fluideinheit, eingestochen zu werden. Auch dieses Injektionselement kann ganz oder teilweise in der Verpackung aufgenommen sein. Als Beispiel einer Anwendung kann ein Auflösen von einer oder mehreren Substanzen genannt werden, beispielsweise von gefriergetrockneten Medikamenten, welche in dem Gefäß aufgenommen sind und mittels mindestens eines durch das Transport- und Transferbehältnis bereitgestellten Lösungsmittels aufgelöst werden. So kann beispielsweise ein Auflösen eines oder mehrerer gefriergetrockneter Medikamente mittels sterilen Wassers (z.B. so genanntem "water for injection") vor einer Anwendung erfolgen.

Das Injektionselement und das Perforationselement können insbesondere miteinander verbunden sein, vorzugsweise fluidisch. So können das Perforationselement und das Injektionselement, wie oben ausgeführt, starr miteinander verbunden sein, beispielsweise indem es sich bei diesen Elementen um einander gegenüber liegende Enden einer Injektionskanüle handelt, welche vorzugsweise beide spitz oder scharf ausgestaltet sind, um die Perforation bzw. die Injektion bewirken zu können. Alternativ oder zusätzlich ist jedoch auch eine andere Art von Verbindung denkbar, beispielsweise eine Schlauchverbindung, eine Verbindung über eine Röhre oder eine andere Art der fluidischen Verbindung. Allgemein kann die Verbindung insbesondere ausgewählt sein aus der Gruppe bestehend aus: einer starren Verbindung, wobei das Perforationselement und das Injektionselement an einander gegenüberliegenden Enden der starren Verbindung ausgebildet sind, beispielsweise in Form einander gegenüber liegender Enden einer beidseitig angespitzten oder angeschärften Injektionsnadel; einer flexiblen Verbindung, insbesondere einer Schlauchverbindung, vorzugsweise einer sterilen Schlauchverbindung.

Das Injektionselement kann insbesondere ein Infusionskit mit mindestens einer Kanüle und mindestens einem Pflaster umfassen. Beispielsweise kann diese Kanüle senkrecht oder zumindest schräg zu einer auf einer Hautoberfläche eines Patienten aufklebbaren Oberfläche des Pflasters angeordnet sein.

Das Transport- und Transferbehältnis kann insbesondere derart ausgestaltet sein, dass ein Herstellen einer Fluidverbindung zwischen dem Injektionselement und dem Innenraum und das Einstechen des Injektionselements in das menschliche oder tierische Körpergewebe und/oder in das von dem Behälter getrennt ausgebildete Gefäß separate Arbeitsschritte bilden.

Das Transport- und Transferbehältnis kann insbesondere mindestens eine Kupplung aufweisen. Die Kupplung kann insbesondere eingerichtet sein, um an mindestens ein von dem Transport- und Transferbehältnis getrennt ausgebildetes Gefäß angekoppelt zu werden. Unter einer Kupplung ist allgemein ein mechanisches Verbindungselement zu verstehen, welches zur Herstellung einer derartigen Verbindung eingerichtet ist, vorzugsweise zur Herstellung einer kraftschlüssigen und/oder formschlüssigen Verbindung. Beispielsweise kann diese Kupplung eine Klemmverbindung, eine Schraubverbindung oder eine Rastverbindung aufweisen. Unter einem Gefäß ist allgemein eine Vorrichtung zu verstehen, welche zur Aufnahme eines oder mehrerer fluider Medien eingerichtet ist, beispielsweise zur Lagerung, zum Transport oder zur Leitung fluider Medien. Insbesondere kann das Gefäß mindestens ein von dem Transport- und Transferbehältnis getrennt ausgebildetes Behältnis und/oder mindestens eine andere Art einer von dem Transport- und Transferbehältnis getrennt ausgebildeten Vorrichtung umfassen. Bei dieser Kupplung soll ein Transfer des fluiden Mediums aus dem Behälter des Transport- und Transferbehältnisses heraus in das angekoppelte Gefäß ermöglicht werden. Beispielsweise kann das Gefäß eine oder mehrere Substanzen enthalten, beispielsweise in fester Form, welche durch das mittels des Transport- und Transferbehältnis bereitgestellte mindestens eine fluide Medium gelöst und/oder suspendiert und/oder emulgiert werden können. Beispielsweise kann das Gefäß mindestens eine gefriergetrocknete Substanz enthalten, beispielsweise mindestens ein gefriergetrocknetes Medikament, welche vor Verwendung aufgelöst werden muss.

Das Transport- und Transferbehältnis kann weiterhin allgemein derart ausgestaltet sein, dass dieses mindestens ein Schaltelement aufweist. Dieses Schaltelement kann insbesondere eingerichtet sein, um in mindestens einer ersten Schalterstellung mittels einer Perforation des perforierbaren Abschnitts die Fluidverbindung zu dem Innenraum des Behälters aufrechtzuerhalten. Weiterhin soll das Schaltelement derart eingerichtet sein, dass dieses in mindestens einer zweiten Schaltstellung die Fluidverbindung unterbricht, vorzugsweise durch Unterbrechung der Perforation des perforierbaren Abschnitts. So kann beispielsweise das Schaltelement als rein mechanisches Schaltelement ausgestaltet sein. Das Schaltelement kann insbesondere direkt oder indirekt auf mindestens ein Perforationselement des Transport- und Transferbehältnisses einwirken, um die Perforation zu bewirken und/oder zu unterbrechen. So kann beispielsweise, wie oben ausgeführt, das Perforationselement mindestens eine Nadel umfassen, insbesondere mindestens eine Kanüle, welche verschiebbar in dem Transport- und Transferbehältnis gelagert sein kann. Das Schaltelement kann beispielsweise eingerichtet sein, um diese Verschiebung zu bewirken und in der ersten Schalterstellung einen perforierten Zustand des perforierbaren Abschnitts bereitzustellen und damit die Fluidverbindung herzustellen, und in mindestens einer zweiten Stellung die Fluidverbindung zu unterbrechen, beispielsweise indem das perforierbare Element aus dem perforierbaren Abschnitt vollständig oder teilweise herausgezogen wird. Der Wechsel zwischen den Schalterstellungen kann insbesondere reversibel erfolgen. So kann beispielsweise das Schaltelement mehrfach zwischen der mindestens einen ersten Schalterstellung und der mindestens einen zweiten Schalterstellung oder umgekehrt hin- und herbewegt werden. Dies kann insbesondere durch eine einfache lineare Einwirkung auf das Perforationselement erfolgen. Das Schaltelement kann insbesondere derart ausgestaltet sein, dass dieses für einen Benutzer von außen her zugänglich ist, ohne dass das Transport- und Transferbehältnis geöffnet werden muss. Beispielsweise kann das Transport- und Transferbehältnis mindestens einen Außenbehälter ausweisen, wobei in dem Außenbehälter der Behälter vollständig oder teilweise angeordnet ist, wobei das Perforationselement vollständig oder teilweise in dem Außenbehälter aufgenommen ist und wobei das Schaltelement eine Relativbewegung zwischen dem Perforationselement und dem Außenbehälter ermöglicht und/oder antreibt. So kann beispielsweise das Perforationselement beweglich, insbesondere verschiebbar, in dem Außenbehälter gelagert sein, wobei diese Bewegung, insbesondere diese Verschiebung, durch das mindestens eine Schaltelement oder einen Teil des selben, beispielsweise einen von außen zugänglichen Griff und/oder Knopf, von einem Benutzer angetrieben werden kann.

Die Energieeinheit kann insbesondere unabhängig von der Perforation des perforierbaren Abschnitts betätigbar sein, um die Beaufschlagung des fluiden Mediums mit dem Überdruck von der Herstellung der Fluidverbindung räumlich und/oder zeitlich zu entkoppeln. Dies kann beispielsweise derart erfolgen, dass die Energieeinheit mechanisch direkt oder indirekt auf den perforierbaren Abschnitt einwirkt, um die Druckbeaufschlagung zu bewirken. Unabhängig hiervon und ohne Beeinflussung dieser Druckbeaufschlagung kann beispielsweise die Perforation des perforierbaren Abschnitts realisierbar sein. Für weitere mögliche Ausgestaltungen kann auf die obige Beschreibung verwiesen werden.

Das Transport- und Transferbehältnis kann insbesondere ganz oder teilweise in mindestens einer Verpackung aufgenommen sein. Für mögliche Ausgestaltungen der Verpackung kann auf die obige Beschreibung verwiesen werden.

Zusammenfassend werden im Rahmen der Erfindung die folgenden Ausführungsformen als besonders bevorzugte Ausgestaltungen vorgeschlagen:
Ausführungsform 1: Herstellungsverfahren zur Herstellung eines Transport- und Transferbehältnisses für mindestens ein fluides Medium, insbesondere für mindestens eine sterile Flüssigkeit, wobei das Verfahren die folgenden Verfahrensschritte umfasst:
   a) Bereitstellen mindestens eines Behälters zur Aufnahme des fluiden Mediums, wobei der Behälter insbesondere ein Verschlusselement aufweisen kann, vorzugsweise mindestens ein perforierbares Verschlusselement und insbesondere einen perforierbaren Stopfen;
   b) Bereitstellen mindestens einer integrierten Energieeinheit, wobei die Energieeinheit eingerichtet ist, um bei einer Verwendung des Transport- und Transferbehältnisses eine - vorzugsweise vollständige - Entleerung des fluiden Mediums aus dem Behälter zu bewirken;
   c) Einbringen des fluiden Mediums in den Behälter und Verschließen des Behälters, insbesondere mit einem verschiebbaren Stopfen;
   d) Beaufschlagen des fluiden Mediums mit einem Überdruck, unter Verwendung der Energieeinheit, wobei der Überdruck bis zu einer Entnahme des fluiden Mediums aufrechtgehalten wird; und
   e) Verpacken zumindest des Behälters in dem mit dem Überdruck beaufschlagten Zustand in mindestens einer Verpackung.

Ausführungsform 2: Herstellungsverfahren nach der vorhergehenden Ausführungsform, wobei nach Durchführung des Verfahrensschritts d) eine Lagerung des Transport- und Transferbehältnisses für eine Zeitdauer von mindestens zwei Wochen, vorzugsweise von mindestens drei Wochen, erfolgt.

Ausführungsform 3: Herstellungsverfahren nach einer der vorhergehenden Ausführungformen, wobei in Schritt c) zunächst mindestens eine Gasblase in dem Behälter erzeugt wird, wobei Schritt d) derart durchgeführt wird, dass die Gasblase nach einer Wartezeit vollständig verschwindet, insbesondere einer Wartezeit von mindestens einer Stunde und besonders bevorzugt nach einer Wartezeit von mindestens zwei Stunden, so dass ein gasblasenfreies Transport- und Transferbehältnis entsteht.

Ausführungsform 4: Herstellungsverfahren nach einer der vorhergehenden Ausführungsformen, wobei die Verfahrensschritte a) bis d) vor einem Vertrieb des Transport- und Transferbehältnisses an einen Kunden, insbesondere einen Endkunden, durchgeführt werden.

Ausführungsform 5: Herstellungsverfahren nach einer der vorhergehenden Ausführungsformen, wobei in Verfahrensschritt d) mindestens ein Federelement des Transport- und Transferbehältnisses gespannt wird, wobei das Federelement auf mindestens einen verschiebbaren Abschnitt des Behälters einwirkt, insbesondere auf mindestens einen Stopfen und besonders bevorzugt auf mindestens einen perforierbaren Stopfen.

Ausführungsform 6: Herstellungsverfahren nach einer der vorhergehenden Ausführungsformen, wobei Verfahrensschritt d) derart durchgeführt wird, dass der Überdruck mindestens ein zu einem Transfer des fluiden Mediums aus dem Behälter heraus erforderliches Druckniveau einnimmt.

Ausführungsform 7: Herstellungsverfahren nach einer der vorhergehenden Ausführungsformen, wobei der Behälter derart ausgestaltet ist, dass das fluide Medium nach Durchführung des Verfahrensschritts d) und vor der Entnahme ausschließlich mit einer Behälterwand des Behälters und einem perforierbaren Abschnitt des Behälters, insbesondere einem perforierbaren Stopfen, in Kontakt steht.

Ausführungsform 8: Herstellungsverfahren nach einer der vorhergehenden Ausführungsformen, wobei der Behälter nach Durchführung des Verfahrensschritts d) ohne Fluidverbindung zwischen einem das fluide Medium aufnehmenden Innenraum des Behälters und einer Umgebung des Behälters ist.

Ausführungsform 9: Herstellungsverfahren nach der vorhergehenden Ausführungsform, wobei der Behälter mindestens einen perforierbaren Abschnitt aufweist, durch welchen hindurch eine Entnahme des fluiden Mediums nach einer Perforation des perforierbaren Abschnitts erfolgen kann.

Ausführungsform 10: Herstellungsverfahren nach einer der vorhergehenden Ausführungsformen, wobei der Behälter und vorzugsweise das Transport- und Transferbehältnis frei von elektrischen Komponenten sind.

Ausführungsform, 11: Herstellungsverfahren nach einer der vorhergehenden Ausführungsformen, wobei nach Durchführung des Verfahrensschritts d) eine Dichtheitskontrolle, insbesondere eine optische Kontrolle, durchgeführt wird, wobei vorzugsweise ein eventuelles Austreten des fluiden Mediums infolge des Überdrucks infolge einer Undichtigkeit des Behälters detektiert wird.

Ausführungsform 12: Herstellungsverfahren nach einer der vorhergehenden Ausführungsformen, wobei nach Durchführung des Verfahrensschritts d) das Transport- und Transferbehältnis in mindestens einer Verpackung aufgenommen wird, insbesondere einem Blisterpack.

Ausführungsform 13: Herstellungsverfahren nach einer der vorhergehenden Ausführungsformen, wobei die Energieeinheit mechanisch mit dem Behälter gekoppelt wird.

Ausführungsform 14: Herstellungsverfahren nach einer der vorhergehenden Ausführungsformen, wobei der Behälter eine Behälterwand mit einer Öffnung umfasst, wobei die Öffnung nach Durchführung des Verfahrensschritts c) durch mindestens ein Element mit einem perforierbaren Abschnitt verschlossen wird, insbesondere durch mindestens einen perforierbaren Stopfen.

Ausführungsform 15: Herstellungsverfahren nach der vorhergehenden Ausführungsform, wobei der Behälter ein röhrenförmiger, einseitig offener Behälter ist.

Ausführungsform 16: Herstellungsverfahren nach einer der beiden vorhergehenden Ausführungsformen, wobei der perforierbare Abschnitt, insbesondere der perforierbare Stopfen, relativ zu der Behälterwand verschiebbar ist, insbesondere parallel zu einer Achse des Behälters.

Ausführungsform, 17: Herstellungsverfahren nach einer der vorhergehenden Ausführungsformen, wobei der Behälter zumindest teilweise in mindestens einem Außenbehälter aufgenommen wird, insbesondere nach Durchführung des Verfahrensschritts c).

Ausführungsform 18: Herstellungsverfahren nach der vorhergehenden Ausführungsform, wobei die Energieeinheit zumindest teilweise in einem Zwischenraum zwischen dem Außenbehälter und dem Behälter angeordnet wird.

Ausführungsform 19: Herstellungsverfahren nach einer der beiden vorhergehenden Ausführungsformen, wobei Verfahrensschritt d) zumindest teilweise während eines Verbindens des Außenbehälters mit dem Behälter und/oder während eines Schließens des Außenbehälters erfolgt, wobei vorzugsweise die Energieeinheit mechanisch gespannt wird.

Ausführungsform 20: Herstellungsverfahren nach einer der vorhergehenden Ausführungsformen, wobei die Energieeinheit eingerichtet ist, um einen Druck auf mindestens einen verschiebbaren Abschnitt des Behälters auszuüben, insbesondere auf einen verschiebbaren perforierbaren Abschnitt und vorzugsweise auf einen perforierbaren verschiebbaren Stopfen.

Ausführungsform 21: Herstellungsverfahren nach einer der vorhergehenden Ausführungsformen, wobei die Energieeinheit eine mechanische Energieeinheit umfasst und insbesondere als eine rein mechanische Energieeinheit ohne elektrische oder elektromechanische Komponenten ausgestaltet ist.

Ausführungsform 22:Herstellungsverfahren nach einer der vorhergehenden Ausführungsformen, wobei die Energieeinheit mindestens ein Federelement umfasst, insbesondere mindestens eine Spiralfeder und/oder mindestens eine Schraubendruckfeder.

Ausführungsform 23: Herstellungsverfahren nach der vorhergehenden Ausführungsform, wobei das Federelement nach Durchführung des Verfahrensschritts d) mindestens einen Teil des Behälters gegen mindestens ein Widerlager des Transport- und Transferbehältnisses presst.

Ausführungsform 24: Herstellungsverfahren nach der vorhergehenden Ausführungsform, wobei das Federelement derart auf den Behälter einwirkt, dass mindestens ein verschiebbarer Abschnitt des Behälters, insbesondere ein verschiebbarer perforierbarer Abschnitt und besonders bevorzugt ein verschiebbarer perforierbarer Stopfen, welcher den Behälter verschließt, gegen das Widerlager gepresst wird.

Ausführungsform 25: Herstellungsverfahren nach einer der beiden vorhergehenden Ausführungsformen, wobei das Widerlager Teil eines den Behälter zumindest teilweise umschließenden Außenbehälters ist.

Ausführungsform 26: Herstellungsverfahren nach einer der drei vorhergehenden Ausführungsformen, wobei das Widerlager während eines Entleerungsvorgangs, bei welchem das fluide Medium aus dem Behälter entnommen wird, relativ zu dem Behälter verschiebbar ist und vorzugsweise in einen Innenraum des Behälters eindringen kann.

Ausführungsform 27: Herstellungsverfahren nach einer der vier vorhergehenden Ausführungsformen, wobei das Widerlager mindestens eine Perforationsöffnung umfasst, wobei mindestens ein Perforationselement, insbesondere mindestens eine Kanüle, durch die Perforationsöffnung zu mindestens einem perforierbaren Abschnitt des Behälters, insbesondere zu mindestens einem perforierbaren Stopfen, vordringen kann.

Ausführungsform 28: Herstellungsverfahren nach einer der vorhergehenden Ausführungsformen, wobei die Energieeinheit eingerichtet ist, um während einer Entnahme des fluiden Mediums im Rahmen einer Verwendung des Transport- und Transferbehältnisses einen im Wesentlichen füllstandsunabhängigen Druck auf das fluide Medium auszuüben.

Ausführungsform 29: Bereitstellungsverfahren zur Bereitstellung eines fluiden Mediums, umfassend:
- eine Herstellung eines Transport- und Transferbehältnisses unter Verwendung eines Herstellungsverfahrens gemäß einer der vorhergehenden Ausführungsformen; und
- eine Perforation mindestens eines perforierbaren Abschnitts zur Herstellung einer Fluidverbindung zwischen einem Innenraum des Behälters und einem Außenraum zum Zweck einer Entnahme des fluiden Mediums aus dem Behälter.

Ausführungsform 30: Bereitstellungsverfahren nach der vorhergehenden Ausführungsform, weiterhin umfassend einen Transfer zumindest eines Teils des fluiden Mediums durch die Fluidverbindung, wobei der Transfer vollständig oder teilweise durch die Energieeinheit angetrieben wird.

Ausführungsform 31: Bereitstellungsverfahren nach der vorhergehenden Ausführungsform, wobei der Transfer vollständig durch eine in Verfahrensschritt d) in der Energieeinheit gespeicherte Energie, insbesondere eine mechanische Energie, bewirkt wird.

Ausführungsform 32: Bereitstellungsverfahren nach einer der beiden vorhergehenden Ausführungsformen, wobei die Bereitstellung zumindest teilweise in eine Injektionskanüle hinein erfolgt.

Ausführungsform 33: Bereitstellungsverfahren nach einer der vorhergehenden, ein Bereitstellungsverfahren betreffenden Ausführungsformen, wobei die Bereitstellung zumindest teilweise in mindestens ein von dem Transport- und Transferbehältnis getrennt ausgebildetes Behältnis erfolgt.

Ausführungsform 34: Bereitstellungsverfahren nach einer der vorhergehenden, ein Bereitstellungsverfahren betreffenden Ausführungsformen, wobei das Verfahren wiederholt durchgeführt wird, wobei nacheinander eine Perforation des perforierbaren Abschnitts mehrerer Behälter zur Herstellung einer Fluidverbindung zwischen einem Innenraum der Behälter und einem Außenraum zum Zweck einer Entnahme des fluiden Mediums aus den Behältern erfolgt, wobei ein Wechsel der Behälter ohne Eintrag von Gasblasen erfolgt.

Ausführungsform 35: Bereitstellungsverfahren nach einer der vorhergehenden, ein Bereitstellungsverfahren betreffenden Ausführungsformen, wobei die Bereitstellung zumindest teilweise in mindestens eine flexible Verbindung, insbesondere in mindestens eine Schlauchverbindung, erfolgt.

Ausführungsform 36: Bereitstellungsverfahren nach der vorhergehenden Ausführungsform, wobei die flexible Verbindung in mindestens einem Infusionskit mündet, wobei das Infusionskit mindestens eine Infusionskanüle und mindestens ein Pflaster umfasst.

Ausführungsform 37: Bereitstellungsverfahren nach einer der vorhergehenden, ein Bereitstellungsverfahren betreffenden Ausführungsformen, weiterhin umfassend mindestens einen Unterbrechungsschritt, wobei in dem Unterbrechungsschritt die Perforation des perforierbaren Abschnitts aufgehoben wird und die Fluidverbindung unterbrochen wird.

Ausführungsform 38: Bereitstellungsverfahren nach einer der vorhergehenden, ein Bereitstellungsverfahren betreffenden Ausführungsformen, wobei die Bereitstellung keinen an einem menschlichen oder tierischen Körper durchgeführten therapeutischen, diagnostischen oder chirurgischen Verfahrensschritt umfasst.

Ausführungsform 39: Transport- und Transferbehältnis zur Lagerung und Bereitstellung mindestens eines fluiden Mediums, insbesondere zur Lagerung und Bereitstellung mindestens einer sterilen Flüssigkeit, wobei das Transport- und Transferbehältnis umfasst:
A) mindestens einen Behälter mit dem darin aufgenommenen fluiden Medium, wobei der Behälter eine Behälterwand und mindestens einen perforierbaren Abschnitt aufweist, insbesondere einen perforierbaren Stopfen, wobei mittels einer Perforation des perforierbaren Abschnitts eine Fluidverbindung zu einem Innenraum des Behälters herstellbar ist zum Zweck einer Entnahme des fluiden Mediums aus dem Behälter;
B) mindestens eine integrierte Energieeinheit, wobei die Energieeinheit eingerichtet ist, um bei einer Verwendung des Transport- und Transferbehältnisses eine - vorzugsweise vollständige - Entleerung des fluiden Mediums aus dem Behälter zu bewirken, wobei die Energieeinheit eingerichtet ist, um das fluide Medium in dem Behälter mit einem Überdruck zu beaufschlagen, wobei der Überdruck bis zu einer Entnahme des fluiden Mediums aufrechtgehalten werden kann,
wobei das Transport- und Transferbehältnis weiterhin mindestens eine Verpackung aufweist, wobei zumindest der Behälter in dem mit dem Überdruck beaufschlagten Zustand in der Verpackung aufgenommen ist.

Ausführungsform 40: Transport- und Transferbehältnis nach der vorhergehenden Ausführungsform, wobei das Transport- und Transferbehältnis herstellbar ist nach einem Herstellungsverfahren gemäß einer der vorhergehenden, ein Herstellungsverfahren betreffenden Ausführungsformen.

Ausführungsform 41: Transport- und Transferbehältnis nach einer der vorhergehenden, ein Transport- und Transferbehältnis betreffenden Ausführungsformen, wobei der perforierbare Abschnitt einen relativ zu einer Behälterwand des Behälters verschiebbar gelagerten Stopfen umfasst und vorzugsweise genau ein relativ zu der Behälterwand verschiebbar gelagerter Stopfen ist.

Ausführungsform 42: Transport- und Transferbehältnis nach einer der vorhergehenden, ein Transport- und Transferbehältnis betreffenden Ausführungsformen, wobei der Behälter ein röhrenförmiger, einseitig mit einer Öffnung versehener Behälter ist.

Ausführungsform 43: Transport- und Transferbehältnis nach einer der vorhergehenden, ein Transport- und Transferbehältnis betreffenden Ausführungsformen, wobei das Transport- und Transferbehältnis und insbesondere der Behälter keine Ventile aufweist.

Ausführungsform 44: Transport- und Transferbehältnis nach einer der vorhergehenden, ein Transport- und Transferbehältnis betreffenden Ausführungsformen, wobei der Behälter vor einer Perforation des perforierbaren Abschnitts ohne Fluidverbindung zwischen einem das fluide Medium aufnehmenden Innenraum des Behälters und einer Umgebung des Behälters ist.

Ausführungsform 45: Transport- und Transferbehältnis nach einer der vorhergehenden, ein Transport- und Transferbehältnis betreffenden Ausführungsformen, wobei die Fluidverbindung ausschließlich durch Perforation des perforierbaren Abschnitts herstellbar ist.

Ausführungsform 46: Transport- und Transferbehältnis nach einer der vorhergehenden, ein Transport- und Transferbehältnis betreffenden Ausführungsformen, wobei die Energieeinheit eingerichtet ist, um den Überdruck nach einer Herstellung des Transport- und Transferbehältnisses während einer Lagerung des Transport- und Transferbehältnisses für eine Zeitdauer von mindestens zwei Wochen, vorzugsweise von mindestens drei Wochen, aufrechtzuerhalten, insbesondere auf einem Druckniveau von mindestens 50 %, vorzugsweise mindestens 70 % und besonders bevorzugt mindestens 80 % eines Anfangsdrucks.

Ausführungsform 47: Transport- und Transferbehältnis nach einer der vorhergehenden, ein Transport- und Transferbehältnis betreffenden Ausführungsformen, wobei die Energieeinheit mindestens ein Federelement umfasst, wobei das Federelement auf den perforierbaren Abschnitt einwirkt.

Ausführungsform 48: Transport- und Transferbehältnis nach einer der vorhergehenden, ein Transport- und Transferbehältnis betreffenden Ausführungsformen, wobei der Behälter und vorzugsweise das Transport- und Transferbehältnis frei von elektrischen Komponenten sind.

Ausführungsform 49: Transport- und Transferbehältnis nach einer der vorhergehenden, ein Transport- und Transferbehältnis betreffenden Ausführungsformen, weiterhin umfassend mindestens einen Außenbehälter, wobei der Behälter zumindest teilweise in dem Außenbehälter aufgenommen ist.

Ausführungsform 50: Transport- und Transferbehältnis nach der vorhergehenden Ausführungsform, wobei die Energieeinheit zumindest teilweise in einem Zwischenraum zwischen dem Außenbehälter und dem Behälter angeordnet ist.

Ausführungsform 51: Transport- und Transferbehältnis nach einer der beiden vorhergehenden Ausführungsformen, wobei der Außenbehälter den Behälter zumindest teilweise röhrenförmig umschließt.

Ausführungsform 52: Transport- und Transferbehältnis nach einer der drei vorhergehenden Ausführungsformen, wobei der Außenbehälter und die Energieeinheit derart eingerichtet sind, dass während eines Verbindens des Außenbehälters mit dem Behälter und/oder während eines Schließens des Außenbehälters die Druckbeaufschlagung des fluiden Mediums erfolgt, wobei vorzugsweise die Energieeinheit mechanisch gespannt wird.

Ausführungsform 53: Transport- und Transferbehältnis nach der vorhergehenden Ausführungsform, wobei der Außenbehälter mindestens zwei miteinander verbindbare Behälterteile aufweist, wobei das Transport- und Transferbehältnis derart eingerichtet ist, dass bei einem Verbinden der Behälterteile die Energieeinheit mechanisch gespannt wird.

Ausführungsform, 54: Transport- und Transferbehältnis nach der vorhergehenden Ausführungsform, wobei das Verbinden der Behälterteile über eine Verbindung erfolgt, ausgewählt aus einer Schraubverbindung und einer Rastverbindung.

Ausführungsform 55: Transport- und Transferbehältnis nach einer der vorhergehenden, ein Transport- und Transferbehältnis betreffenden Ausführungsformen wobei die Energieeinheit eine rein mechanische Energieeinheit ist, ohne elektrische und/oder elektromechanische Komponenten, vorzugsweise eine manuell bedienbare aber automatisch spannbare Energieeinheit.

Ausführungsform 56: Transport- und Transferbehältnis nach einer der vorhergehenden, ein Transport- und Transferbehältnis betreffenden Ausführungsformen, wobei die Energieeinheit mindestens ein Federelement umfasst, insbesondere mindestens eine Spiralfeder und/oder mindestens eine Schraubendruckfeder.

Ausführungsform, 57: Transport- und Transferbehältnis nach der vorhergehenden Ausführungsform, wobei das Federelement eingerichtet ist, um mindestens einen Teil des Behälters, insbesondere den perforierbaren Abschnitt und besonders bevorzugt den perforierbaren Stopfen, gegen mindestens ein Widerlager des Transport- und Transferbehältnisses zu pressen.

Ausführungsform, 58: Transport- und Transferbehältnis nach der vorhergehenden Ausführungsform, wobei das Federelement derart auf den Behälter einwirkt, dass mindestens ein verschiebbarer perforierbarer Stopfen gegen das Widerlager gepresst wird.

Ausführungsform 59: Transport- und Transferbehältnis nach einer der beiden vorhergehenden Ausführungsformen, wobei das Widerlager Teil eines den Behälter zumindest teilweise umschließenden Außenbehälters ist.

Ausführungsform 60: Transport- und Transferbehältnis nach einer der drei vorhergehenden Ausführungsformen, wobei das Widerlager während eines Entleerungsvorgangs, bei welchem das fluide Medium aus dem Behälter entnommen wird, relativ zu dem Behälter verschiebbar ist und vorzugsweise in einen Innenraum des Behälters eindringen kann.

Ausführungsform 61: Transport- und Transferbehältnis nach einer der vier vorhergehenden Ausführungsformen, wobei das Widerlager mindestens eine Perforationsöffnung umfasst, wobei mindestens ein Perforationselement, insbesondere mindestens eine Kanüle, durch die Perforationsöffnung zu dem perforierbaren Abschnitt des Behälters, insbesondere zu mindestens einem perforierbaren Stopfen, vordringen kann.

Ausführungsform 62: Transport- und Transferbehältnis nach der vorhergehenden Ausführungsform, wobei das Perforationselement Bestandteil des Transport- und Transferbehältnisses ist.

Ausführungsform 63: Transport- und Transferbehältnis nach der vorhergehenden Ausführungsform, wobei das Perforationselement verschiebbar in dem Transport- und Transferbehältnis gelagert ist, insbesondere verschiebbar in einem Außenbehälter.

Ausführungsform 64: Transport- und Transferbehältnis nach einer der vorhergehenden, ein Transport- und Transferbehältnis betreffenden Ausführungsformen, wobei die Energieeinheit eingerichtet ist, um während einer Entnahme des fluiden Mediums im Rahmen einer Verwendung des Transport- und Transferbehältnisses einen im Wesentlichen Füllstands-unabhängigen Druck auf das fluide Medium auszuüben.

Ausführungsform 65: Transport- und Transferbehältnis nach einer der vorhergehenden, ein Transport- und Transferbehältnis betreffenden Ausführungsformen, wobei das Transport- und Transferbehältnis mindestens ein Perforationselement zur Perforation des perforierbaren Abschnitts umfasst, insbesondere eine Kanüle.

Ausführungsform 66: Transport- und Transferbehältnis nach einer der vorhergehenden, ein Transport- und Transferbehältnis betreffenden Ausführungsformen, weiterhin umfassend mindestens ein Injektionselement, insbesondere eine Injektionskanüle, wobei das Injektionselement eingerichtet ist, um in ein menschliches oder tierisches Körpergewebe und/oder in ein von dem Behälter getrennt ausgebildetes Gefäß eingestochen zu werden.

Ausführungsform 67: Transport- und Transferbehältnis nach den beiden vorhergehenden Ausführungsformen, wobei das Perforationselement und das Injektionselement miteinander verbunden sind.

Ausführungsform, 68: Transport- und Transferbehältnis nach der vorhergehenden Ausführungsform, wobei die Verbindung ausgewählt ist aus der Gruppe bestehend aus:
- einer starren Verbindung, wobei das Perforationselement und das Injektionselement an einander gegenüberliegenden Enden der starren Verbindung ausgebildet sind;
- einer flexiblen Verbindung, insbesondere einer Schlauchverbindung, vorzugsweise einer sterilen Schlauchverbindung.

Ausführungsform 69: Transport- und Transferbehältnis nach einer der beiden vorhergehenden Ausführungsformen, wobei das Injektionselement ein Infusionskit mit einer Kanüle und einem Pflaster umfasst.

Ausführungsform 70: Transport- und Transferbehältnis nach einer der vier vorhergehenden Ausführungsformen, wobei das Transport- und Transferbehältnis derart ausgestaltet ist, dass ein Herstellen einer Fluidverbindung zwischen dem Injektionselement und dem Innenraum und das Einstechen des Injektionselements in das menschliche oder tierische Körpergewebe und/oder in das von dem Behälter getrennt ausgebildete Gefäß separate Arbeitsschritte bilden.

Ausführungsform 71: Transport- und Transferbehältnis nach einer der vorhergehenden, ein Transport- und Transferbehältnis betreffenden Ausführungsformen, wobei das Transport- und Transferbehältnis mindestens eine Kupplung aufweist, wobei die Kupplung eingerichtet ist, um an mindestens ein von dem Transport- und Transferbehältnis getrennt ausgebildetes Gefäß angekoppelt zu werden, insbesondere an mindestens ein von dem Transport- und Transferbehältnis getrennt ausgebildetes Behältnis und/oder an mindestens eine von dem Transport- und Transferbehältnis getrennt ausgebildete Vorrichtung, wobei ein Transfer des fluiden Mediums aus dem Behälter des Transport- und Transferbehältnisses in das angekoppelte Gefäß ermöglicht wird.

Ausführungsform 72: Transport- und Transferbehältnis nach einer der vorhergehenden, ein Transport- und Transferbehältnis betreffenden Ausführungsformen, wobei das Transport- und Transferbehältnis mindestens ein Schaltelement aufweist, wobei das Schaltelement eingerichtet ist, um in mindestens einer ersten Schalterstellung mittels einer Perforation des perforierbaren Abschnitts die Fluidverbindung zu dem Innenraum des Behälters aufrechtzuerhalten, wobei das Schaltelement weiterhin eingerichtet ist, um in mindestens einer zweiten Schalterstellung die Fluidverbindung zu unterbrechen, vorzugsweise durch Unterbrechung der Perforation des perforierbaren Abschnitts.

Ausführungsform 73: Transport- und Transferbehältnis nach einer der vorhergehenden, ein Transport- und Transferbehältnis betreffenden Ausführungsformen, wobei die Energieeinheit unabhängig von der Perforation des perforierbaren Abschnitts betätigbar ist, um die Beaufschlagung des fluiden Mediums mit dem Überdruck von der Herstellung der Fluidverbindung zeitlich zu entkoppeln.

Ausführungsform 74: Transport- und Transferbehältnis nach einer der vorhergehenden, ein Transport- und Transferbehältnis betreffenden Ausführungsformen, wobei das fluide Medium blasenfrei in dem Behältnis aufgenommen ist.

Ausführungsform 75: Transport- und Transferbehältnis nach einer der vorhergehenden, ein Transport- und Transferbehältnis betreffenden Ausführungsformen, wobei ein Druckniveau in dem fluiden Medium und eine Lagerzeit derart bestimmt sind, dass sich keine Gasblase in dem Behältnis befindet.

Ausführungsform 76: Transport- und Transferbehältnis nach einer der vorhergehenden, ein Transport- und Transferbehältnis betreffenden Ausführungsformen, wobei ein Druckniveau in dem fluiden Medium ausreichend ist, um eine anfänglich vorhandene Gasblase in dem fluiden Medium über die Zeit, insbesondere über eine Zeitdauer von höchstens einem Monat und besonders bevorzugt von höchstens einer Woche, vollständig aufzulösen.

Ausführungsform 77: Transport- und Transferbehältnis nach einer der vorhergehenden, ein Transport- und Transferbehältnis betreffenden Ausführungsformen, wobei das Transport- und Transferbehältnis derart eingerichtet ist, dass das fluide Medium während eines Transports und einer Lagerung des Transport- und Transferbehältnisses ausschließlich mit einer Behälterwand des Behälters und einem Verschlusselement des Behälters, insbesondere einem Stopfen, in Berührung kommt.

Die im Rahmen der vorliegenden Erfindung vorgeschlagenen Verfahren und Vorrichtungen weisen gegenüber bekannten Verfahren und Vorrichtungen der genannten Art zahlreiche Vorteile auf. So befinden sich weltweit zahlreiche Injektions- und Transfersysteme für sterile Flüssigkeiten im Einsatz. Dabei wird in der Regel die Flüssigkeit nach Abfüllung drucklos, d.h. unter Atmosphärendruck, gelagert. Erst bei oder während der Anwendung wird der Druck in der Flüssigkeit erhöht, um einen Transfer durchzuführen. Spritzen haben dabei schon konstruktionsbedingt von Anfang an eine Fluidverbindung für den späteren Transfer. Weiterhin existieren so genannte Carpulen, welche ein Septum aufweisen, über das eine Fluidverbindung hergestellt werden kann. Zudem existieren sogenannte Injektionspens, bei denen zuerst eine Fluidverbindung hergestellt und dann der Druck erhöht wird. Weiterhin sind auch Sonderformen von Spritzen und Carpulen denkbar, bei denen die Fluidverbindung erst direkt bei der Anwendung und zusammen mit der Druckerhöhung hergestellt wird. Wie oben bei der Beschreibung des Standes der Technik bereits ausgeführt, weisen derartige Systeme jedoch allgemein verschiedene Nachteile auf, die im Rahmen der vorliegenden Erfindung durch die oben beschriebenen bevorzugten Merkmale auf einfache und zuverlässige Weise überwunden werden können. Während beispielsweise Spritzen mit vorhandener Fluidverbindung in der Regel mindestens drei Komponenten aufweisen, nämlich den zylinderförmigen Körper, den verschiebbaren Stopfen und die Abdeckung der Fluidverbindung, welche in Wechselwirkung mit dem Produkt stehen, kann das erfindungsgemäße Transport- und Transferbehältnis derart ausgestaltet werden, dass der Behälter zunächst ohne Fluidverbindung hergestellt wird. Als einfachste Form des Behälters ist eine einseitig geschlossene Röhre möglich, welche nach dem Befüllen mit einem verschiebbaren Stopfen verschlossen wird. Andere Ausführungsformen können beispielsweise Carpulen und Röhren mit zwei verschiebbaren Stopfen sein. Neben Behältern mit einer starren Behälterwand, beispielsweise aus einem Glasmaterial und/oder einem Kunststoffmaterial, sind Behälter mit formbarer oder flexibler Wand denkbar, welche nicht notwendigerweise vollständig aus einem formsteifen und/oder elastischen Material bestehen müssen.

Das vorgeschlagene Transport- und Transferbehältnis, bei welchem das fluide Medium mit einem Überdruck beaufschlagt ist, weist insbesondere bei hochviskosen fluiden Medien Vorteile auf Insbesondere lassen sich auf diese Weise hochviskose Medikamente dosieren und/oder injizieren, welche mittels herkömmlicher Spritzen nicht mehr injiziert werden können.
Ein weiterer Vorteil besteht darin, dass bei herkömmlichen Systemen bei der Abfüllung entstehende Luftblasen in der Regel später nicht mehr entfernbar sind und/oder erst vom Anwender manuell entfernt werden. Im Gegensatz hierzu kann erfindungsgemäß das fluide Medium in dem Behälter über die Energieeinheit, welche eine oder mehrere Energieträger unterschiedlicher Art umfassen kann, unter einen Überdruck gesetzt werden, wobei das gewählte Druckniveau über dem erforderlichen oder gewünschten Transferdruck für den Transfer des fluiden Mediums aus dem Innenraum des Behälters heraus liegen sollte. Beispielsweise kann die Energieeinheit eine oder mehrere Federelemente zur Druckerhöhung aufweisen, welche technisch einfach realisierbar sind. Das mindestens eine Federelement kann beispielsweise mindestens eine rein mechanische Feder umfassen oder auch eine oder mehrere Gasfedern und/oder eine oder mehrere Flüssigkeits-/Gas-Federn. Ein positiver Nebeneffekt stellt sich mit der Druckerhöhung ein, bei der durch die erhöhte Gaslöslichkeit vorhandene Blasen im Behälter verschwinden können. Idealerweise wird der Druck schon beim Hersteller erhöht und bleibt bis zur Verwendung auf dem zur Injektion und/oder dem Transfer der Flüssigkeit aus dem Behälter erforderlichen Niveau. Somit besitzt das Transport- und Transferbehältnis vorzugsweise eine integrierte, systembedingte Dichtigkeitskontrolle. Sowohl bei der Herstellung als auch vom Anwender kann eine Integrität des Transport- und Transferbehältnisses auf einfache Weise überprüft werden. Eine Dichtigkeitskontrolle kann insbesondere auch vom Anwender durchgeführt werden, beispielsweise vor einer Verwendung, da beispielsweise eine Undichtigkeit ein Entleeren des Behälters nach längerer Lagerzeit beinhalten kann.

Ein Transfer des fluiden Mediums aus dem Innenraum des Behälters heraus kann auf einfache Weise realisiert werden, insbesondere ausschließlich durch Herstellung der Fluidverbindung, vorzugsweise durch eine Perforation des perforierbaren Abschnitts des Behälters.

Der Behälter mit der Energieeinheit kann auch als eigenständige Baugruppe ohne Injektionsteil hergestellt und vertrieben werden. Dabei werden das optionale Transfersystem und/oder das optionale Injektionssystem erst beim Anwender aus mehreren Baugruppen zusammengestellt, beispielsweise aus dem Transport- und Transferbehältnis sowie einer weiteren Injektorbaugruppe.

Das Transport- und Transferbehältnis kann somit Bestandteil eines Injektors sein oder kann selbst als Injektor ausgestaltet werden. Beispielsweise kann es sich hierbei um einen Autoinjektor handeln. Autoinjektoren weisen eine wachsende Bedeutung auf, da in der Praxis in der Regel ein Trend zu einer Selbstmedikation besteht. Autoinjektoren werden beispielsweise für kleinere Volumina bis hin zu 1 ml eingesetzt. Dementsprechend kann der Behälter beispielsweise eingerichtet sein, um eine Menge an fluidem Medium von 0,1-1,0 ml aufzunehmen. Auch andere Flüssigkeitsmengen sind jedoch grundsätzlich möglich, beispielsweise Flüssigkeitsmengen von 0,5 ml - 200 ml, beispielsweise von 1 ml - 100 ml. Größere Flüssigkeitsmengen sind insbesondere dadurch realisierbar, dass in einer bevorzugten Ausgestaltung die Perforation des mindestens einen perforierbaren Abschnitts des Behälters reversibel ist, sodass beispielsweise ein Transfer des fluiden Mediums aus dem Behälter heraus unterbrochen und optional anschließend wieder aufgenommen werden kann. Somit besteht eine hohe Flexibilität hinsichtlich der Ausgestaltung des Transferprozesses, beispielsweise hinsichtlich transferierter Fluidmengen, Transferdauern, eine Abbruch des Transfers und anderer Optionen.

Bei bekannten Autoinjektoren sticht die Nadel des Injektors in der Regel in das Körpergewebe ein, und die Flüssigkeit wird durch den Druckaufbau in der Spritze oder Carpule verdrängt. Neben Autoinjektoren gibt es auch wiederverwendbare Injektionssysteme wie beispielsweise Insulinpumpen, welche größere Flüssigkeitsmengen zum Teil portionsweise über lange Zeit injizieren. Auch bei derartigen Systemen wird üblicherweise der für den Transfer benötige Druck erst im Augenblick des Transfers aufgebaut. Derartige bekannte Systeme wie Autoinjektoren oder Medikationspumpen sind jedoch zur Injektion von größeren Flüssigkeitsmengen in der Regel ungünstig, da beispielsweise der Autoinjektor über die gesamte Injektionsdauer an der Injektionsstelle verbleiben muss. Außerdem sind die Anwender in der Wahl der Injektionsstellen, abhängig von der Beweglichkeit, in der Regel stark eingeschränkt. Eine optische Kontrolle über den Fortgang und Abschluss der Injektion ist in vielen Fällen erschwert.

Mittels des erfindungsgemäßen Transport- und Transferbehältnisses kann hingegen das Grundprinzip bekannter Autoinjektoren auf zwei Funktionsgruppen verteilt werden, nämlich zum einen die Funktionsgruppe, welche den Behälter, die Energieeinheit und den Auslösemechanismus aufweist, wobei letzterer beispielsweise das mindestens eine Perforationselement umfassen kann, und eine zweite Gruppe, welche für eine Injektion in das Gewebe zuständig ist. Letztere Gruppe kann beispielsweise, wie oben beschrieben, mindestens ein Infusionskit umfassen. Beide Funktionsgruppen können starr oder flexibel miteinander verbunden sein, letzteres beispielsweise über mindestens eine Schlauchverbindung.

Für den Anwender ergibt sich hieraus eine Vielzahl von Vorteilen. So kann eine Injektion in das Körpergewebe beispielsweise unabhängig von dem eigentlichen Einstich und/oder unabhängig von einem Transfer des fluiden Mediums aus dem Behälter heraus erfolgen. Die eigentliche Injektion in das Körpergewebe kann auch an schwer einsehbaren und schlecht kontrollierbaren Stellen erfolgen, wobei das Transport- und Transferbehältnis optional in einem für den Anwender sichtbaren Bereich angeordnet sein kann. Beispielsweise kann das Transport- und Transferbehältnis vollständig oder teilweise transparent ausgestaltet sein, beispielsweise indem der Behälter und vorzugsweise auch der Außenbehälter derart transparent ausgestaltet sind, dass der Benutzer beispielsweise einen Füllstand des Behälters überprüfen kann.

Das Transport- und Transferbehältnis und optional der Injektor können während der Injektion bewegt werden, was insbesondere für lange Injektionszeiten angenehm sein kann. Weiterhin kann der Injektor vollständig oder teilweise gut eingesehen werden, und der Injektionsverlauf kann besser kontrolliert werden. Optional kann das Transport- und Transferbehältnis, wie oben beschrieben, eine Start/Stopp-Funktion enthalten, beispielsweise durch ein entsprechendes Schaltelement. Die Injektion kann in einer oder auch in mehreren Stufen erfolgen. Weiterhin kann das Transport- und Transferbehältnis derart ausgestaltet sein, dass dieses vollständig auf elektrische Antriebe verzichten kann.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigen:
- Figur 1: ein erstes Ausführungsbeispiels eines Transport- und Transferbehältnisses;
- Figur 2: ein zweites Ausführungsbeispiel eines Transport- und Transferbehältnisses mit einer Kupplung zur Ankopplung an ein Gefäß;
- Figur 3: ein drittes Ausführungsbeispiel eines Transport- und Transferbehältnisses mit einem Injektionselement; und
- Figur 4: ein viertes Ausführungsbeispiel eines Transport- und Transferbehältnisses mit einem Schaltelement und einer optionalen Ankopplung an eine Schlauchverbindung.

### Ausführungsbeispiele

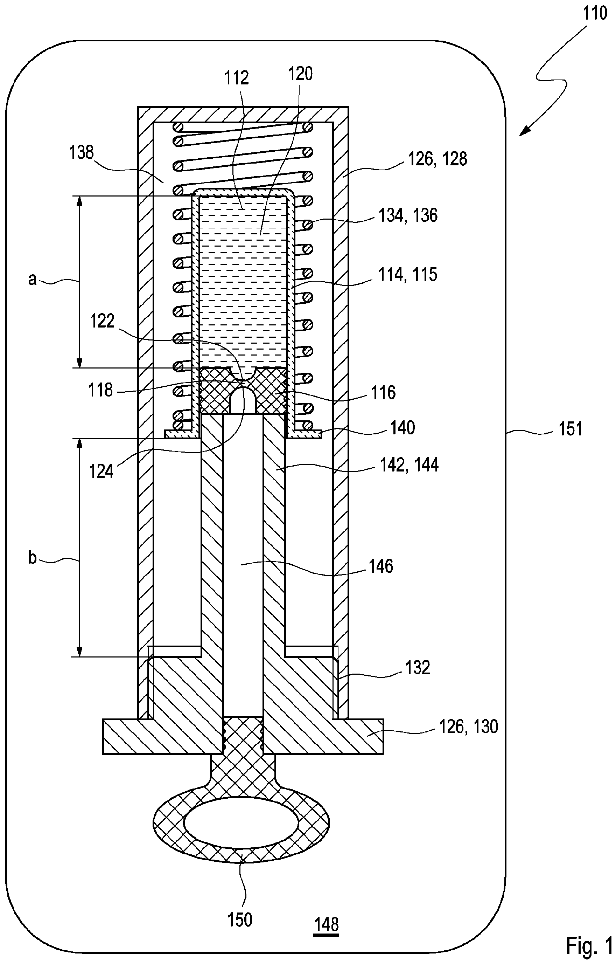

In Figur 1 ist ein erstes Ausführungsbeispiel eines erfindungsgemäßen Transport- und Transferbehältnisses 110 für ein fluides Medium 112, insbesondere eine sterile Flüssigkeit, dargestellt. Das Transport- und Transferbehältnis 110 umfasst in dem dargestellten Ausführungsbeispiel einen Behälter 114, welcher in dem dargestellten Ausführungsbeispiel als einseitig geschlossener, röhrenförmiger Behälter ausgestaltet ist und in der Darstellung gemäß Figur 1 nach unten geöffnet ist. Der Behälter 114 nimmt das fluide Medium 112 auf. Der Behälter 114 weist eine Behälterwand 115 auf, welche beispielsweise ganz oder teilweise aus Glas und/oder einem Kunststoffmaterial hergestellt sein kann. Beispielsweise kann die Behälterwand ganz oder teilweise transparent ausgestaltet sein, so dass das fluide Medium 112 von außen erkennbar ist. Die Behälterwand 115 kann beispielsweise im Wesentlichen röhrenförmig ausgestaltet sein, beispielsweise in Form einer einseitig geschlossenen und vorzugsweise zylindrischen Röhre. Der Behälter 114 ist durch ein Verschlusselement in Form eines beweglichen, perforierbaren Stopfens 116 verschlossen, welcher einen perforierbaren Abschnitt 118 des Behälters 114 bildet. Der Stopfen 116 stellt ein mögliches Ausführungsbeispiel eines Verschlusselements dar, welches den Behälter 114 verschließt. Beispielsweise kann der Stopfen 116 im Bereich dieses perforierbaren Abschnitts 118 eine zu einem Innenraum 120 des Behälters 114 hin weisende Aussparung 122 und/oder eine von dem Innenraum 120 wegweisende Aussparung 124 aufweisen, sodass im Bereich des perforierbaren Abschnitts 118 der Stopfen 116 geschwächt ist und dass, beispielsweise durch die Aussparung 122, eine zumindest weitgehend vollständige Entleerung des Innenraums 120 bei einer bestimmungsgemäßen Verwendung des Transport- und Transferbehältnisses 110 erleichtert wird.

Der Behälter 114 ist in verschlossenem Zustand in dem in Figur 1 dargestellten Ausführungsbeispiel vorzugsweise in einem Außenbehälter 126 aufgenommen. Der Außenbehälter 126 ist vorzugsweise ebenfalls zumindest teilweise transparent ausgestaltet, sodass durch den Außenbehälter 126 hindurch beispielsweise ein Füllstand des Behälters 114 beobachtbar ist. Der Außenbehälter 126 weist optional in dem dargestellten Ausführungsbeispiel zwei Behälterteile 128, 130 auf, welche in dem dargestellten Ausführungsbeispiel in Form eines Behälterteils 128, das als Hohlzylinder ausgestaltet ist, und in Form eines Behälterteils 130, das als Deckelteil ausgestaltet ist, dargestellt sind. Auch andere Ausgestaltungen sind jedoch grundsätzlich möglich. Die Behälterteile 128, 130 sind vorzugsweise über ein oder mehrere Verbindungselemente 132 des Behälterteils 128 und/oder des Behälterteils 130 miteinander verbindbar, sodass der Außenbehälter 126 beispielsweise verschließbar ist.

Weiterhin umfasst das Transport- und Transferbehältnis 110 eine Energieeinheit 134, die vorzugsweise einen Energiespeicher umfassen kann, insbesondere einen mechanischen und/oder hydraulischen Energiespeicher, vorzugsweise in Form eines oder mehrerer Federelemente 136. Beispielsweise kann die Energieeinheit 134 ganz oder teilweise in einem Zwischenraum 138 zwischen dem Außenbehälter 126 und dem Behälter 114 angeordnet sein. Das Federelement kann beispielsweise, wie in Figur 1 gezeigt, an seinem einen Ende auf dem Außenbehälter 126 abgestützt sein und an einem gegenüberliegenden Ende an dem Behälter 114, beispielsweise an einem umlaufenden Rand 140 des Behälters 114, wie in Figur 1 ebenfalls dargestellt.

Die Energieeinheit 134 kann, wie in Figur 1 gezeigt, optional derart ausgestaltet sein, dass der Behälter 114 mit dem Stopfen 116 gegen ein Widerlager 142 gepresst wird. Dieses Widerlager 142 ist in dem dargestellten Ausführungsbeispiel exemplarisch als Bestandteil des Außenbehälters 126 ausgestaltet, insbesondere als Bestandteil des Behälterteils 130. Beispielsweise kann das Widerlager 142 in Form eines Stempels 144 ausgestaltet sein, welcher in einen Innenraum 120 des Behälters 114 hineinragt und auf den Stopfen 116 einwirkt. Auf diese oder auf andere Weise kann mittels der Energieeinheit 134 ein Druck auf das in dem Behälter 114 befindliche fluide Medium 112 ausgeübt werden, welcher während einer Lagerdauer des Transport- und Transferbehältnisses 110 aufrechtgehalten werden kann. Der Stopfen 116 und/oder das Widerlager 142 können somit, in Zusammen- wirkung mit dem Federelement 136, ebenfalls als Bestandteile der Energieeinheit 134 aufgefasst werden.

Beispielsweise kann bei der Herstellung des Transport- und Transferbehältnisses 110 zunächst ein Befüllen und Verschließen des Behälters 114 erfolgen, woraufhin der Behälter 114 in dem Außenbehälter 126 aufgenommen wird. Durch Verbinden der Behälterteile 128, 130 werden die Energieeinheit 134 und das Federelement 136, welches als Energiespeicher wirken kann, gespannt und der Außenbehälter 126 verschlossen.

Das Widerlager 142 kann insbesondere, wie in Figur 1 gezeigt, derart ausgestaltet sein, dass durch dieses Widerlager 142 hindurch eine Perforation des perforierbaren Abschnitts 118 erfolgen kann. Zu diesem Zweck kann das Widerlager beispielsweise mindestens eine Perforationsöffnung 146 umfassen, welche in dem dargestellten Ausführungsbeispiel gemäß Figur 1 exemplarisch als langgestreckter Perforationskanal ausgebildet ist, der sich durch das Widerlager 142 hindurch erstreckt und welcher beispielsweise parallel zu einer Längsachse des röhrenförmigen Behälters 114 ausgebildet ist. Auf einer Außenseite des Außenbehälters 126, welcher einem Außenraum 148 zuweist, kann die Perforationsöffnung 146 optional durch mindestens ein Verschlusselement 150 verschlossen sein, beispielsweise mindestens einen weiteren Stopfen. Das Verschlusselement 150 kann insbesondere derart ausgestaltet sein, dass dieses von einem Benutzer manuell geöffnet werden kann, beispielsweise durch eine in Figur 1 angedeutete Lasche.

Zur bestimmungsgemäßen Verwendung des Transport- und Transferbehältnisses 110 kann optional zunächst das Verschlusselement 150 entfernt werden. Anschließend kann mit einem Perforationselement, welches in Figur 1 nicht dargestellt ist, beispielsweise einer Kanüle, durch die Perforationsöffnung 146 hindurch der Stopfen 116 und dessen perforierbarer Abschnitt 118 perforiert werden, wobei eine Fluidverbindung zu dem Innenraum 120 hergestellt wird. Dieses Herstellen der Fluidverbindung erfolgt vollständig unabhängig von Druckbeaufschlagung des fluiden Mediums. Durch das Perforationselement, beispielsweise eine Kanüle, kann dann der Transfer des fluiden Mediums 112 heraus aus dem Innenraum 120 in den Außenraum 148 oder in eine nicht näher dargestellte Fluidvorrichtung erfolgen. Optional kann die Perforation nach teilweiser Entleerung des Behälters 114 wieder aufgehoben werden, wobei vorzugsweise die Fluidverbindung wieder geschlossen wird.

Weiterhin sind in Figur 1 symbolisch Wegstrecken a und b angedeutet. Für eine vollständige Entleerung des Behälters 114 sollte die Wegstrecke b größer oder zumindest gleich der Wegstrecke a sein. Vorzugsweise ist die Behälterwand 115 in ihrer Form derart ausgestaltet, dass auch bei einem maximalen Eintauchen des Stopfens 116 in den Innenraum 120 keine nicht entleerbaren Totvolumina entstehen, was beispielsweise durch einen flachen Boden des Behälters 114 realisiert werden kann.

Weiterhin ist das Transport- und Transferbehältnis 110 derart ausgestaltet, dass Teile desselben, zumindest der Behälter 114 sowie optional ein oder mehrere weitere Bestandteile, ganz oder teilweise in einer Verpackung 151 aufgenommen sind. Diese Verpackung 151 ist in Figur 1 lediglich symbolisch angedeutet und kann beispielsweise eine Kunststoffverpackung, insbesondere eine Folienverpackung, umfassen. Die Verpackung 151 ist im Sinne der Erfindung begrifflich selbst Bestandteil des Transport- und Transferbehältnisses 110, auch wenn diese beispielsweise vor einem bestimmungsgemäßen Gebrauch geöffnet und/oder ganz oder teilweise entfernt werden kann. Die übrigen Bestandteile des Transport- und Transferbehältnisses 110, mit Ausnahme der Verpackung 151, können beispielsweise eine oder mehrere Nutzungseinheiten bilden, welche für einen Transfer des fluiden Mediums 112 aus dem Innenraum 120 heraus genutzt werden können. Beispielsweise können zumindest der Behälter 114 und optional die Energieeinheit 134 in der Verpackung 151 aufgenommen sein, in einem Zustand, in welchem das fluide Medium 112 durch die Energieeinheit 134 mit dem Überdruck beaufschlagt ist.

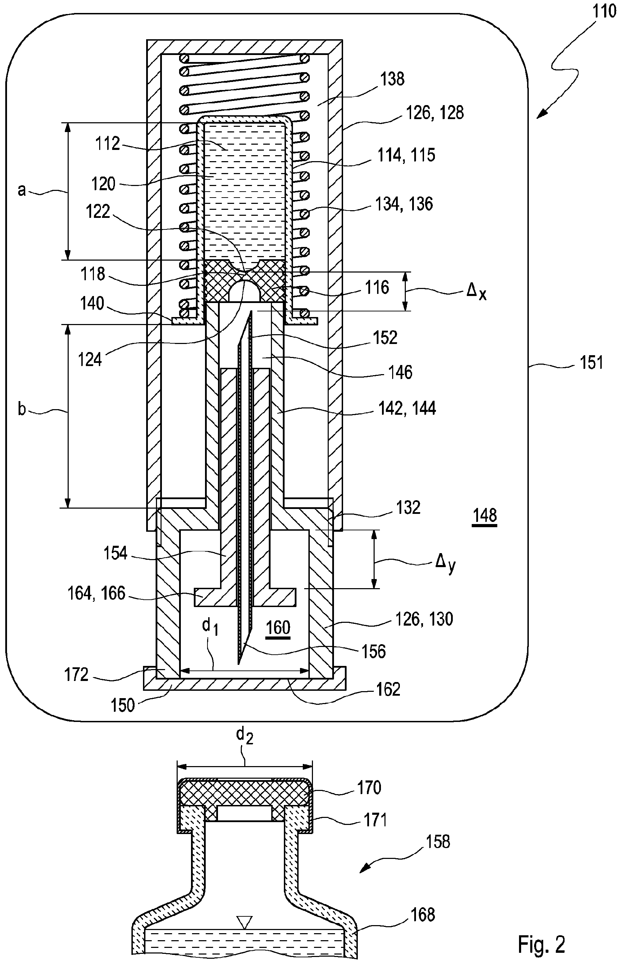

In Figur 2 ist ein zu Figur 1 alternatives Ausführungsbeispiel eines Transport- und Transferbehältnisses 110 dargestellt, wiederum, analog zu Figur 1, in einer Schnittdarstellung. Das Transport- und Transferbehältnis 110 kann zunächst wiederum analog zu Figur 1 ausgestaltet sein und beispielsweise einen das fluide Medium 112 aufnehmenden Behälter 114 mit einem Stopfen 116 aufweisen. Dieser Stopfen 116 kann wiederum analog zu Figur 1 ausgestaltet sein und kann beispielsweise wiederum Aussparungen 122, 124 im Bereich eines perforierbaren Abschnitts 118 aufweisen. Bezüglich möglicher Ausgestaltungen kann auf die obige Beschreibung verwiesen werden. Wiederum ist weiterhin auch ein Außenbehälter 126 vorgesehen, welcher wiederum optional mehrere Behälterteile, hier zwei Behälterteile 128, 130, aufweisen kann. Diese Behälterteile 128, 130 können beispielsweise wiederum über eine oder mehrere Verbindungselemente 132 miteinander verbunden sein, beispielsweise wiederum über eine oder mehrere Schraubverbindungen.

In einem Zwischenraum 138 zwischen dem Außenbehälter 126 und dem Behälter 114 ist wiederum eine Energieeinheit 134 vorgesehen, welche beispielsweise wiederum einen Energiespeicher in Form beispielsweise eines Federelements 136 umfassen kann. Auch diesbezüglich kann wiederum auf die obige Beschreibung der Figur 1 verwiesen werden.

Das Federelement 136 kann beispielsweise wiederum auf einem Rand 140 des Behälters 114 abgestützt sein.

Wiederum umfasst in dem dargestellten Ausführungsbeispiel der Außenbehälter 126 ein Widerlager 142, welches beispielsweise Bestandteil des Behälterteils 130 sein kann und welches auf den Stopfen 116 einwirken kann. Diesbezüglich kann auf die obige Beschreibung der Figur 1 verwiesen werden. Das Widerlager 142 kann beispielsweise wiederum als Stempel 144 ausgestaltet sein und umfasst wiederum eine Perforationsöffnung 146.

In dem dargestellten Ausführungsbeispiel ist weiterhin in dem Außenbehälter 126, insbesondere vollständig oder teilweise innerhalb der Perforationsöffnung 146, ein Perforationselement 152 vorgesehen, beispielsweise in Form einer dem Stopfen 116 zuweisenden Kanüle. Dieses Perforationselement 152 kann beispielsweise beweglich innerhalb des Außenbehälters 126 gelagert sein, derart, dass dieses eine Perforationsbewegung zur Perforation des perforierbaren Abschnitts 118 durchführen kann, in Figur 2 nach oben gerichtet. Das Perforationselement 152 kann beispielsweise in einem Führungselement 154 aufgenommen sein, welches seinerseits beweglich in der Perforationsöffnung 146 gelagert sein kann. Alternativ oder zusätzlich kann das Perforationselement 152 auch direkt in der Perforationsöffnung 146 gelagert sein. Verschiedene Ausgestaltungen sind möglich.

In dem dargestellten Ausführungsbeispiel weist das Transport- und Transferbehältnis weiterhin optional ein Injektionselement 156 auf. Dieses Injektionselement 156, welches beispielsweise wiederum eine Kanüle umfassen kann, kann zum Transfer des fluiden Mediums 112 in ein von dem Transport- und Transferbehältnis getrennt ausgebildetes Gefäß 158 eingerichtet sein und/oder zu einer Injektion des fluiden Mediums 112 in ein Körpergewebe. Das Gefäß 158 kann beispielsweise ein Behältnis sein, in welchem optional beispielsweise ein gefriergetrocknetes Pulver aufgenommen sein kann, welches vor einer weiteren Verwendung aufgelöst werden soll, beispielsweise durch ein mittels des Transport-und Transferbehältnis 110 bereitgestelltes Lösungsmittel. Das Injektionselement 156 ist fluidisch verbunden mit dem Perforationselement 152, so dass durch das Perforationselement 152 und durch das Injektionselement 156 ein Fluidtransfer des fluiden Mediums 112 ermöglicht wird.

Das Perforationselement 152 und das Injektionselement 156 können beispielsweise, wie in Figur 2 gezeigt, starr miteinander verbunden sein oder auch über eine flexible Verbindung, beispielsweise eine Schlauchverbindung. Die starre Verbindung kann beispielsweise dadurch realisiert werden, dass das Perforationselement 152 und das Injektionselement 156 einander gegenüberliegende Enden einer Injektionskanüle bilden, welche auf beiden Seiten mit einer Spitze, einer Schneide oder einer ähnlichen Einrichtung verbunden ist.

Das Injektionselement 156 ragt in dem in Figur 2 dargestellten Ausführungsbeispiel exemplarisch in einen Hohlraum 160 des Außenbehälters 126 hinein, welcher, wie in Figur 2 gezeigt, von dem Zwischenraum 138 getrennt ausgestaltet sein kann oder welcher auch ganz oder teilweise mit diesem identisch ausgebildet sein kann. Dieser Hohlraum 160 kann beispielsweise durch ein Verschlusselement 150, beispielsweise eine Kappe, verschließbar sein, um das Injektionselement 156 zu schützen. Das Führungselement 154, welches vorzugsweise starr mit dem Perforationselement 152 und/oder dem Injektionselement 156 verbunden ist, kann beispielsweise an seinem einer Öffnung 162 des Hohlraums 160 zuweisenden Ende eine Aufweitung 164 aufweisen, welche eine Bedienbarkeit des Führungselements 154 ermöglicht. Optional kann das Führungselement 154 auch von außen bedienbar sein, sodass eine Perforationsbewegung beispielsweise manuell lenkbar ist. In diesem Fall kann die Aufweitung 164 beispielsweise durch eine Aussparung in dem Behälterteil 130 von außen zugänglich sein und/oder kann nach außen, in einen Außenraum 148 ragen, sodass die Aufweitung 164 auch als Schaltement 166 dienen kann, um die Perforation des perforierbaren Abschnitts 118 zu steuern. Die Aufweitung 164 sollte derart weit von einem Beginn der Perforationsöffnung 146 und/oder von einem Anschlag für die Aufweitung 164 entfernt sein (Abstand Δy in Figur 2) dass diese Aufweitung 164 sich um mindestens eine Strecke nach oben bewegen kann, welche einem Abstand zwischen der Spitze des Perforationselements 152 und dem Innenraum 120 des Behälters 114 (Abstand Δx in Figur 2) entspricht. Auf diese Weise ist vorzugsweise sichergestellt, dass sich zum Zweck einer Perforationsbewegung das Perforationselement 152 mindestens soweit nach oben bewegen kann, dass der Stopfen 116 vollständig perforiert und eine Fluidverbindung durch den Stopfen 116 hindurch zwischen dem Innenraum 120 und dem Außenraum 148 hergestellt werden kann. Weiterhin sind in Figur 2 wiederum, analog zu Figur 1, die Wegstrecken a und b eingezeichnet. Für eine maximale Entleerung sollte wiederum die Wegstrecke b größer oder zumindest gleich der Wegstrecke a sein.

Beim der von dem Transport- und Transferbehältnis 110 getrennt ausgebildeten Gefäß 158 kann es sich beispielsweise, wie in Figur 2 gezeigt, um eine Phiole 168 und/oder um ein anderes Gefäß, insbesondere Glasgefäß, handeln, welche beispielsweise an ihrem oberen Ende mit einem Septum 170 verschlossen sein kann. Das Septum 170 kann beispielsweise durch eine in Figur 2 ebenfalls angedeutete Bördelkappe 171 oder eine andere Art von Halteelement fixiert werden. Auch andere Ausgestaltungen des Gefäßes 158 sind jedoch grundsätzlich möglich. Das Transport- und Transferbehältnis 110 kann beispielsweise eine oder mehrere Kupplungen 172 umfassen, welche eine mechanische Ankopplung an das Gefäß 158 ermöglichen. In dem dargestellten Ausführungsbeispiel kann die Kupplung 172 beispielsweise einen Kragen umfassen, welcher beispielsweise von seinem Innendurchmesser her an einen Außendurchmesser des Septums 170 und/oder eines Halses der Phiole 168 angepasst ist. Dies ist in Figur 2 durch die Durchmesser d₁ und d₂ angedeutet, wobei gelten sollte: d₁ ≥ d₂ oder d₁ > d₂.

Beispielsweise kann ein Transfer des fluiden Mediums 112 aus dem Innenraum 120 des Behälters 114 heraus in das Gefäß 158 derart erfolgen, dass zunächst optional das Verschlusselement 150 entfernt wird. Anschließend erfolgt die Ankopplung des Gefäßes 158, indem das Septum 170 mit seiner Oberseite auf die Aufweitung 164 des Führungselements 154 gepresst wird. Dabei perforiert das Injektionselement 156 das Septum 170, und gleichzeitig wird das Führungselement 164 in Figur 2 nach oben geschoben. Hierdurch perforiert, zeitgleich oder zeitlich versetzt, das Perforationselement 152 den perforierbaren Abschnitt 118 des Stopfens 116, und die Fluidverbindung zu dem Innenraum 120 wird hergestellt. Durch den Überdruck, mit welchem das fluide Medium 112 beaufschlagt ist, wird das fluide Medium 112 durch das Perforationselement 152 und das Injektionselement 156 hindurch durch das Septum 170 in das Gefäß 158 transferiert. In diesem Gefäß 158 können sich bereits eine oder mehrere Flüssigkeiten befinden. Insgesamt ist auf diese Weise ein steriler Transfer des fluiden Mediums 112 möglich.

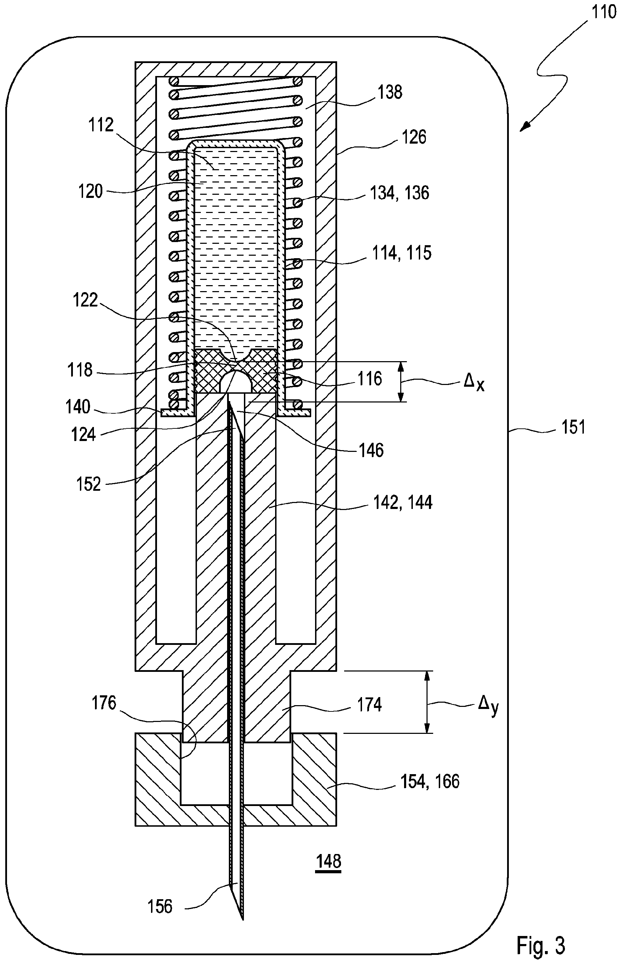

In Figur 3 ist ein weiteres Ausführungsbeispiel eines erfindungsgemäßen Transport- und Transferbehältnisses 110 in einer zu den Figuren 1 und 2 analogen Darstellung gezeigt. Das Transport- und Transferbehältnis 110 kann zunächst analog zu dem Ausführungsbeispiel gemäß Figur 2 ausgestaltet sein, sodass weitgehend auf die obige Beschreibung der Figur 2 verwiesen werden kann. Wiederum ist ein Widerlager 142 vorgesehen, gegen welches mittels der Energieeinheit 134 der Stopfen 116 gepresst wird, analog zu dem Beispiel in den Figuren 1 und 2. Der Außenbehälter 126 ist, im Gegensatz zu den Ausführungsbeispielen in den Figuren 1 und 2, hier einteilig dargestellt, kann jedoch grundsätzlich wiederum auch mehrteilig ausgestaltet sein, beispielsweise durch ein oder mehrere Verbindungselemente 132 analog zu den Figuren 1 und 2, sodass beispielsweise auch ein Anpressdruck des Widerlagers 142 gegen den Stopfen 116 eingestellt werden kann.

Im Unterschied zu dem Ausführungsbeispiel gemäß Figur 2 ist weiterhin zwar auch wiederum ein Perforationselement 152 teilweise in einer Perforationsöffnung 146 des Widerlagers 142 aufgenommen. Dieses ist jedoch in dem dargestellten Ausführungsbeispiel gemäß Figur 3 nicht in einem Führungselement 154 aufgenommen sondern direkt in der Perforationsöffnung 146 gelagert. Auch eine Ausgestaltung mit einem zusätzlichen Führungselement 154, analog beispielsweise zur Ausgestaltung gemäß Figur 2, ist jedoch grundsätzlich möglich.

Das Perforationselement 152 ist wiederum beispielsweise als durchgängige Kanüle ausgestaltet, an deren dem Perforationselement 152 gegenüberliegenden Ende beispielsweise wiederum ein Injektionselement 156 aufgenommen sein kann. Dieses Injektionselement 156 kann beispielsweise in dem dargestellten Ausführungsbeispiel aus dem Transport- und Transferbehältnis 110 herausragen. Das Perforationselement 152 und das Injektionselement 156 können beispielsweise wiederum fluidisch miteinander verbunden sein, beispielsweise durch die Kanüle.

Zwar ist in dem Ausführungsbeispiel gemäß Figur 3 kein in der Perforationsöffnung 146 angeordnetes Führungselement 154 vorgesehen, jedoch ein Führungselement 154, welches außerhalb dieser Perforationsöffnung 146 angeordnet ist. Dieses Führungselement 154 kann beispielsweise auch wiederum als Schaltelement 166 dienen, beispielsweise um manuell oder wiederum beispielsweise durch Anpressen an ein Septum 170 eine Perforation des Stopfens 116 zu bewirken. Hierzu kann das Schaltelement 166 beispielsweise in Figur 3 nach oben geschoben werden, wobei beispielsweise ein Fortsatz 174 an dem Außenbehälter 126 in eine Aussparung 176 in dem Schaltelement 166 eingreifen kann, sodass das Schaltelement 166 in seiner Bewegung geführt ist. In Figur 3 ist somit, analog zur Darstellung gemäß Figur 2, eine zweite Schalterstellung des Schaltelements 166 dargestellt, bei welcher der Stopfen 116 nicht perforiert ist und somit keine Fluidverbindung zu dem Innenraum 120 hergestellt ist. Wird hingegen das Schaltelement 166 in Figur 3 wie auch in Figur 2 nach oben bewegt, so dringt das Perforationselement 152 durch den Stopfen 116 ein, und die Fluidverbindung wird hergestellt. Dann befindet sich das Schaltelement 166 in einer ersten Schalterstellung, wobei in dieser ersten Schalterstellung die Fluidverbindung besteht. Vorzugsweise ist dieses Herstellen der Fluidverbindung reversibel, sodass das Schaltelement 166 auch wieder aus der ersten Schaltposition in die zweite Schaltposition überführbar ist, beispielsweise indem dieses Schaltelement 166 in den Figuren 2 und 3 wieder nach unten gezogen wird. Das Betätigen des Schaltelements 166 kann manuell oder auch automatisch erfolgen, beispielsweise bei Aufsetzen des Transport- und Transferbehältnisses 110 auf mindestens ein Gefäß 158 und/oder während eines Injektionsvorgangs. Auch eine Mischform der in den Figuren 2 und 3 dargestellten Ausführungsbeispiele ist möglich, beispielsweise indem das Führungselement 154 in dem Ausführungsbeispiel gemäß Figur 3 einen Fortsatz aufweist, welcher in die Perforationsöffnung 146 eingreift und dort geführt wird.

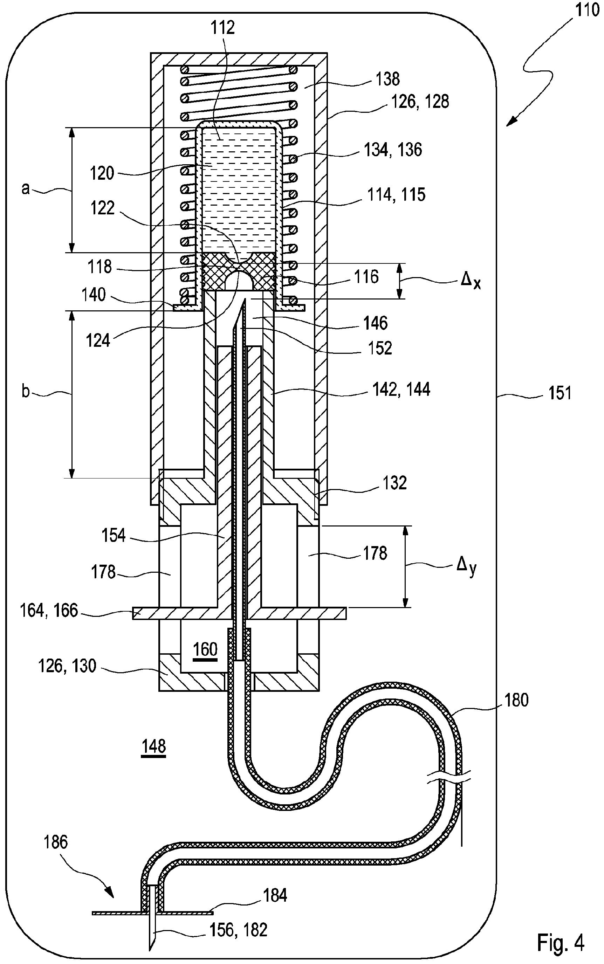

In Figur 4 ist eine weitere Abwandlung des Ausführungsbeispiels des Transport- und Transferbehältnisses 110 gemäß Figur 2 gezeigt. Diese Ausgestaltung entspricht zunächst wiederum weitgehend der Ausgestaltung gemäß Figur 2, sodass weitgehend auf die obige Beschreibung verwiesen werden kann. Im Unterschied zum Ausführungsbeispiel gemäß Figur 2 ist in dem dargestellten Ausführungsbeispiel gemäß Figur 4 das Schaltelement 166 aus dem Hohlraum 160 des Behälterteils 130 herausgeführt, sodass dieses bequem vom Außenraum 148 her bedienbar ist. Auf diese Weise kann das Schaltelement 166 beispielsweise von der in Figur 4 dargestellten Schalterstellung in eine Schalterstellung transferiert werden, in welcher das Perforationselement 152 den perforierbaren Abschnitt 118 des Stopfens 116 perforiert, in dem das Schaltelement 166 in Figur 1 nach oben geführt wird. Um das Schaltelement 166 aus dem Hohlraum 160 herauszuführen, sind in der Wand des Hohlraums 160 eine oder mehrere Öffnungen 178 vorgesehen.

Ein weiterer Unterschied zum Ausführungsbeispiel gemäß Figur 2 besteht darin, dass das Perforationselement 152 nicht starr mit einem Injektionselement 156 verbunden ist. Stattdessen ist ein Injektionselement 156 vorgesehen, welches mit dem Perforationselement 152 fluidisch über eine flexible Verbindung 180 verbunden ist, insbesondere über eine flexible, sterile Schlauchverbindung. Diese flexible Verbindung 180 kann beispielsweise in dem Hohlraum 160 oder bereits in dem Außenraum 148 mit einem Ende an ein dem Perforationselement 152 gegenüberliegendes Ende einer Kanüle angekoppelt sein. An einem gegenüberliegenden Ende kann die flexible Verbindung 180 mit dem Injektionselement 156 verbunden werden. Dieses Injektionselement 156 kann beispielsweise, wie in dem Ausführungsbeispiel gemäß Figur 4 dargestellt, als Infusionskanüle 182 ausgestaltet sein, welche mitsamt einem Pflaster 184 zum Aufbringen auf eine Haut eines Patienten Bestandteil eines Infusionskits 186 ist. Auch andere Ausgestaltungen sind jedoch grundsätzlich möglich.

Mittels der in Figur 4 dargestellten Anordnung kann ein Infusionsvorgang auf einfache und zuverlässige Weise durchgeführt werden. So kann beispielsweise ein Einstechen des Injektionselements 156 in ein Körpergewebe eines Benutzers mechanisch vollständig entkoppelt von einer Herstellung der Fluidverbindung durch den Stopfen 116 hindurch erfolgen. Beispielsweise kann zunächst das Einstechen des Injektionselements 156 und ein Aufkleben des Pflasters 184 auf die Hautoberfläche des Benutzers erfolgen. Anschließend kann beispielsweise durch Betätigen des Schaltelements 166 die Fluidverbindung hergestellt werden, sodass die Infusion starten kann. Diese Infusion kann auch wieder reversibel unterbrochen werden, ebenfalls wiederum durch Betätigen des Schaltelements 166. Alternativ kann auch bereits vor einem Einstechen des Injektionselements 156 in die Hautoberfläche die flexible Verbindung 180 bereits vollständig oder teilweise mit dem fluiden Medium 112 befüllt werden, beispielsweise indem ein erstes Mal das Schaltelement 166 betätigt wird. Anschließend kann die Fluidverbindung zu dem Innenraum 120 durch Betätigen des Schaltelements 166 wieder unterbrochen werden, das Injektionselement 156 kann in das Körpergewebe eingestochen werden, und das Schaltelement 166 kann anschließend erneut betätigt werden, um die eigentliche Infusion zu starten. Auf diese Weise kann eine blasenfreie Injektion durchgeführt werden.

**Bezugszeichenliste**

| | | | |
|---|---|---|---|
| 110 | Transport- und Transferbehältnis | 150 | Verschlusselement |
| 112 | fluides Medium | 151 | Verpackung |
| 114 | Behälter | 152 | Perforationselement |
| 115 | Behälterwand | 154 | Führungselement |
| 116 | Stopfen | 156 | Injektionselement |
| 118 | perforierbarer Abschnitt | 158 | Gefäß |
| 120 | Innenraum | 160 | Hohlraum |
| 122 | Aussparung | 162 | Öffnung |
| 124 | Aussparung | 164 | Aufweitung |
| 126 | Außenbehälter | 166 | Schaltelement |
| 128 | Behälterteil | 168 | Phiole |
| 130 | Behälterteil | 170 | Septum |
| 132 | Verbindungselement | 171 | Bördelkappe |
| 134 | Energieeinheit | 172 | Kupplung |
| 136 | Federelement | 174 | Fortsatz |
| 138 | Zwischenraum | 176 | Aussparung |
| 140 | Rand | 178 | Öffnung |
| 142 | Widerlager | 180 | flexible Verbindung |
| 144 | Stempel | 182 | Infusionskanüle |
| 146 | Perforationsöffnung | 184 | Pflaster |
| 148 | Außenraum | 186 | Infusionskit |

## Patentansprüche

1. Herstellungsverfahren zur Herstellung eines Transport- und Transferbehältnisses (110) für mindestens ein fluides Medium (112), insbesondere für mindestens eine sterile Flüssigkeit, wobei das Verfahren die folgenden Verfahrensschritte umfasst:
a) Bereitstellen mindestens eines Behälters (114) zur Aufnahme des fluiden Mediums (112);
b) Bereitstellen mindestens einer Energieeinheit (134), wobei die Energieeinheit (134) eingerichtet ist, um bei einer Verwendung des Transport- und Transferbehältnisses (110) eine Entleerung des fluiden Mediums (112) aus dem Behälter (114) zu bewirken, insbesondere eine vollständige Entleerung,
c) Einbringen des fluiden Mediums (112) in den Behälter (114) und Verschließen des Behälters (114) mit einem verschiebbaren und perforierbaren Stopfen (116),
d) Beaufschlagen des fluiden Mediums (112) mit einem Überdruck unter Verwendung der Energieeinheit (134), wobei die Energieeinheit (134) mindestens ein Federelement (136) umfasst, wobei der Überdruck bis zu einer Entnahme des fluiden Mediums (112) aufrechtgehalten wird, und
e) Verpacken zumindest des Behälters (114) in dem mit dem Überdruck beaufschlagten Zustand in mindestens einer Verpackung (151).

2. Herstellungsverfahren nach dem vorhergehenden Anspruch, wobei in Schritt c) zunächst mindestens eine Gasblase in dem Behälter (114) erzeugt wird, wobei Schritt d) derart durchgeführt wird, dass die Gasblase nach einer Wartezeit vollständig verschwindet, insbesondere einer Wartezeit von mindestens einer Stunde und besonders bevorzugt nach einer Wartezeit von mindestens zwei Stunden, so dass ein gasblasenfreies Transport- und Transferbehältnis (110) entsteht.

3. Herstellungsverfahren nach einem der vorhergehenden Ansprüche, wobei in Verfahrensschritt d) mindestens ein Federelement (136) des Transport- und Transferbehältnisses (110) gespannt wird, wobei das Federelement (136) auf mindestens einen verschiebbaren Abschnitt des Behälters (114) einwirkt, insbesondere auf mindestens einen Stopfen (116) und besonders bevorzugt auf mindestens einen perforierbaren Stopfen (116).

4. Herstellungsverfahren nach einem der vorhergehenden Ansprüche, wobei der Behälter (114) unmittelbar nach Durchführung des Verfahrensschritts d) ohne Fluidverbindung zwischen einem das fluide Medium (112) aufnehmenden Innenraum (120) des Behälters (114) und einer Umgebung (148) des Behälters (114) ist, wobei der Behälter (114) mindestens einen perforierbaren Abschnitt (118) aufweist, durch welchen hindurch eine Entnahme des fluiden Mediums (112) nach einer Perforation des perforierbaren Abschnitts (118) erfolgen kann.

5. Herstellungsverfahren nach einem der vorhergehenden Ansprüche, wobei das Federelement (136) nach Durchführung des Verfahrensschritts d) mindestens einen Teil des Behälters (114) gegen mindestens ein Widerlager (142) des Transport- und Transferbehältnisses (110) presst, wobei das Widerlager (142) mindestens eine Perforationsöffnung (146) umfasst, wobei mindestens ein Perforationselement (152) durch die Perforationsöffnung (146) zu mindestens einem perforierbaren Abschnitt (118) des Behälters (114) vordringen kann.

6. Bereitstellungsverfahren zur Bereitstellung mindestens eines fluiden Mediums (112), umfassend:
- eine Herstellung eines Transport- und Transferbehältnisses (110) unter Verwendung eines Herstellungsverfahrens gemäß einem der vorhergehenden Ansprüche; und
- eine Perforation mindestens eines perforierbaren Abschnitts (118) zur Herstellung einer Fluidverbindung zwischen einem Innenraum (120) des Behälters (114) und einem Außenraum zum Zweck einer Entnahme des fluiden Mediums (112) aus dem Behälter (114).

7. Bereitstellungsverfahren nach dem vorhergehenden Anspruch, weiterhin umfassend mindestens einen Unterbrechungsschritt, wobei in dem Unterbrechungsschritt die Perforation des perforierbaren Abschnitts (118) aufgehoben wird und die Fluidverbindung unterbrochen wird.

8. Transport- und Transferbehältnis (110) zur Lagerung und Bereitstellung mindestens eines fluiden Mediums (112), insbesondere zur Lagerung und Bereitstellung mindestens einer sterilen Flüssigkeit, wobei das Transport- und Transferbehältnis (110) umfasst:
A) mindestens einen Behälter (114) mit dem darin aufgenommenen fluiden Medium (112), wobei der Behälter (114) eine Behälterwand (115) und mindestens einen perforierbaren Abschnitt (118) in Form eines verschiebbaren Stopfens (116) aufweist, wobei mittels einer Perforation des perforierbaren Abschnitts (118) eine Fluidverbindung zu einem Innenraum (120) des Behälters (114) herstellbar ist zum Zweck einer Entnahme des fluiden Mediums (112) aus dem Behälter (114);
B) mindestens eine integrierte Energieeinheit (134), wobei die Energieeinheit (134) eingerichtet ist, um bei einer Verwendung des Transport- und Transferbehältnisses (110) eine Entleerung des fluiden Mediums (112) aus dem Behälter (114) zu bewirken, vorzugsweise eine vollständige Entleerung, wobei die Energieeinheit (134) mindestens ein Federelement (136) umfasst, wobei die Energieeinheit (134) eingerichtet ist, um das fluide Medium (112) in dem Behälter (114) mit einem Überdruck zu beaufschlagen, wobei der Überdruck bis zu einer Entnahme des fluiden Mediums (112) aufrechtgehalten werden kann,
wobei das Transport- und Transferbehältnis (110) weiterhin mindestens eine Verpackung (151) aufweist, wobei zumindest der Behälter (114) in dem mit dem Überdruck beaufschlagten Zustand in der Verpackung (151) aufgenommen ist.

9. Transport- und Transferbehältnis (110) nach dem vorhergehenden Anspruch, weiterhin umfassend mindestens einen Außenbehälter (126), wobei der Behälter (114) zumindest teilweise in dem Außenbehälter (126) aufgenommen ist, wobei der Außenbehälter (126) und die Energieeinheit (134) derart eingerichtet sind, dass während eines Verbindens des Außenbehälters (126) mit dem Behälter (114) und/oder während eines Schließens des Außenbehälters (126) die Druckbeaufschlagung des fluiden Mediums (112) erfolgt, wobei vorzugsweise die Energieeinheit (134) mechanisch gespannt wird.

10. Transport- und Transferbehältnis (110) nach einem der vorhergehenden, ein Transport- und Transferbehältnis (110) betreffenden Ansprüche, wobei das Federelement (136) insbesondere mindestens eine Schraubendruckfeder umfasst, wobei das Federelement (136) eingerichtet ist, um mindestens einen Teil des Behälters (114), insbesondere den perforierbaren Abschnitt (118) und besonders bevorzugt einen perforierbaren Stopfen (116), gegen mindestens ein Widerlager (142) des Transport- und Transferbehältnisses (110) zu pressen.

11. Transport- und Transferbehältnis (110) nach einem der vorhergehenden, ein Transport- und Transferbehältnis (110) betreffenden Ansprüche, wobei das Transport- und Transferbehältnis (110) mindestens ein Perforationselement (152) zur Perforation des perforierbaren Abschnitts (118) umfasst.

12. Transport- und Transferbehältnis (110) nach einem der vorhergehenden, ein Transport- und Transferbehältnis (110) betreffenden Ansprüche, weiterhin umfassend mindestens ein Injektionselement (156), wobei das Injektionselement (156) eingerichtet ist, um in ein menschliches oder tierisches Körpergewebe und/oder ein von dem Behälter (114) getrennt ausgebildetes Gefäß (158) eingestochen zu werden.

13. Transport- und Transferbehältnis (110) nach dem vorhergehenden Anspruch, wobei das Transport- und Transferbehältnis (110) derart ausgestaltet ist, dass ein Herstellen einer Fluidverbindung zwischen dem Injektionselement (156) und dem Innenraum (120) und das Einstechen des Injektionselements (156) in das menschliche oder tierische Körpergewebe und/oder in das von dem Behälter (114) getrennt ausgebildete Gefäß (158) separate Arbeitsschritte bilden.

14. Transport- und Transferbehältnis (110) nach einem der vorhergehenden, ein Transport- und Transferbehältnis (110) betreffenden Ansprüche, wobei das Transport- und Transferbehältnis (110) mindestens ein Schaltelement (166) aufweist, wobei das Schaltelement (166) eingerichtet ist, um in mindestens einer ersten Schalterstellung mittels einer Perforation des perforierbaren Abschnitts (118) die Fluidverbindung zu dem Innenraum (120) des Behälters (114) aufrechtzuerhalten, wobei das Schaltelement (166) weiterhin eingerichtet ist, um in mindestens einer zweiten Schalterstellung die Fluidverbindung zu unterbrechen, vorzugsweise durch Unterbrechung der Perforation des perforierbaren Abschnitts (118).

15. Transport- und Transferbehältnis (110) nach einem der vorhergehenden, ein Transport- und Transferbehältnis (110) betreffenden Ansprüche, wobei die Energieeinheit (134) eingerichtet ist, um auf den perforierbaren Abschnitt (118) mechanisch einzuwirken.

## Claims

1. Production method for producing a transport and transfer receptacle (110) for at least one fluid medium (112), in particular for at least one sterile liquid, wherein the method comprises the following method steps:
a) supplying at least one container (114) for receiving the fluid medium (112);
b) supplying at least one energy unit (134), wherein the energy unit (134) is configured to effect emptying of the fluid medium (112) from the container (114), in particular complete emptying, when the transport and transfer receptacle (110) is used,
c) introducing the fluid medium (112) into the container (114) and closing the container (114) with a displaceable and perforable stopper (116),
d) charging the fluid medium (112) with positive pressure while using the energy unit (134), wherein the energy unit (134) comprises at least one spring element (136), wherein the positive pressure is maintained until the fluid medium (112) is removed, and
e) packing at least the container (114) in the state charged with positive pressure in at least one packaging (151).

2. Production method according to the preceding claim, wherein, in step c), first of all at least one gas bubble is produced in the container (114), wherein step d) is carried out in such a manner that the gas bubble completely disappears after a waiting period, in particular a waiting period of at least one hour and, particularly preferably, after a waiting period of at least two hours, thus resulting in a transport and transfer receptacle (110) which is free from gas bubbles.

3. Production method according to one of the preceding claims, wherein, in method step d), at least one spring element (136) of the transport and transfer receptacle (110) is tensioned, wherein the spring element (136) acts on at least one displaceable section of the container (114), in particular on at least one stopper (116) and, particularly preferably, on at least one perforable stopper (116).

4. Production method according to one of the preceding claims, wherein, directly after method step d) has been carried out, the container (114) does not have a fluid connection between an interior (120) of the container (114), the interior receiving the fluid medium (112), and a surroundings (148) of the container (114), wherein the container (114) has at least one perforable section (118) through which the fluid medium (112) can be removed after the perforable section (118) is perforated.

5. Production method according to one of the preceding claims, wherein, after method step d) has been carried out, the spring element (136) presses at least part of the container (114) against at least one abutment (142) of the transport and transfer receptacle (110), wherein the abutment (142) comprises at least one perforation opening (146), wherein at least one perforating element (152) can penetrate through the perforation opening (146) to at least one perforable section (118) of the container (114).

6. Supply method for supplying at least one fluid medium (112), comprising:
- producing a transport and transfer receptacle (110) using a production method according to one of the preceding claims; and
- perforating at least one perforable section (118) in order to produce a fluid connection between an interior (120) of the container (114) and an exterior for the purpose of removing the fluid medium (112) from the container (114).

7. Supply method according to the preceding claim, furthermore comprising at least one interruption step, wherein, in the interruption step, the perforating of the perforable section (118) is stopped and the fluid connection is interrupted.

8. Transport and transfer receptacle (110) for storing and supplying at least one fluid medium (112), in particular for storing and supplying at least one sterile liquid, wherein the transport and transfer receptacle (110) comprises:
A) at least one container (114) with the fluid medium (112) received therein, wherein the container (114) has a container wall (115) and at least one perforable section (118) in the form of a displaceable stopper (116), wherein, by perforating the perforable section (118), a fluid connection to an interior (120) of the container (114) can be produced for the purpose of removing the fluid medium (112) from the container (114);
B) at least one integrated energy unit (134), wherein the energy unit (134) is configured in order to effect emptying of the fluid medium (112) from the container (114), preferably complete emptying, when the transport and transfer receptacle (110) is used, wherein the energy unit (134) comprises at least one spring element (136), wherein the energy unit (134) is configured in order to charge the fluid medium (112) in the container (114) with positive pressure, wherein the positive pressure can be maintained until the fluid medium (112) is removed,
wherein the transport and transfer receptacle (110) furthermore has at least one packaging (151), wherein at least the container (114) in the state charged with positive pressure is received in the packaging (151).

9. Transport and transfer receptacle (110) according to the preceding claim, furthermore comprising at least one external container (126), wherein the container (114) is at least partially accommodated in the external container (126), wherein the external container (126) and the energy unit (134) are configured in such a manner that the fluid medium (112) is pressurized during connection of the external container (126) to the container (114) and/or during closing of the external container (126), wherein the energy unit (134) is preferably mechanically tensioned.

10. Transport and transfer receptacle (110) according to one of the preceding claims relating to a transport and transfer receptacle (110), wherein the spring element (136) comprises in particular at least one helical compression spring, wherein the spring element (136) is configured in order to press at least part of the container (114), in particular the perforable section (118) and, particularly preferably, a perforable stopper (116), against at least one abutment (142) of the transport and transfer receptacle (110).

11. Transport and transfer receptacle (110) according to one of the preceding claims relating to a transport and transfer receptacle (110), wherein the transport and transfer receptacle (110) comprises at least one perforating element (152) for perforating the perforable section (118).

12. Transport and transfer receptacle (110) according to one of the preceding claims relating to a transport and transfer receptacle (110), furthermore comprising at least one injection element (156), wherein the injection element (156) is configured in order to be inserted into a human or animal body tissue and/or a vessel (158) formed separately from the container (114).

13. Transport and transfer receptacle (110) according to the preceding claim, wherein the transport and transfer receptacle (110) is designed in such a manner that producing a fluid connection between the injection element (156) and the interior (120) and inserting the injection element (156) into the human or animal body tissue and/or into the vessel (158) formed separately from the container (114) form separate working steps.

14. Transport and transfer receptacle (110) according to one of the preceding claims relating to a transport and transfer receptacle (110), wherein the transport and transfer receptacle (110) has at least one switching element (166), wherein the switching element (166) is configured in order, in at least one first switch position, to maintain the fluid connection to the interior (120) of the container (114) by perforating the perforable section (118), wherein the switching element (166) is furthermore configured in order, in at least one second switch position, to interrupt the fluid connection, preferably by interrupting the perforating of the perforable section (118).

15. Transport and transfer receptacle (110) according to one of the preceding claims relating to a transport and transfer receptacle (110), wherein the energy unit (134) is configured in order to act mechanically on the perforable section (118).

## Revendications

1. Procédé de fabrication pour la fabrication d'un récipient de transport et de transfert (110) pour au moins un milieu fluide (112), en particulier pour au moins un liquide stérile, dans lequel le procédé comprend les étapes de procédé suivantes:
a) préparer au moins un réservoir (114) destiné à contenir le milieu fluide (112);
b) préparer au moins une source d'énergie (134), dans lequel la source d'énergie (134) est conçue pour provoquer une vidange du milieu fluide (112) hors du réservoir (114) lors d'une utilisation du récipient de transport et de transfert (110), en particulier une vidange complète,
c) introduire le milieu fluide (112) dans le réservoir (114) et fermer le réservoir (114) avec un bouchon déplaçable et perforable (116),
d) soumettre le milieu fluide (112) à une surpression en utilisant la source d'énergie (134), dans lequel la source d'énergie (134) comprend au moins un élément de ressort (136), dans lequel la surpression est maintenue jusqu'à un prélèvement du milieu fluide (112), et
e) emballer au moins le réservoir (114) dans l'état soumis à la surpression dans au moins un emballage (151).

2. Procédé de fabrication selon la revendication précédente, dans lequel à l'étape c) on produit d'abord au moins une bulle de gaz dans le réservoir (114), dans lequel on exécute l'étape d) de telle manière que la bulle de gaz disparaisse complètement après un temps d'attente, en particulier un temps d'attente d'au moins une heure et de préférence après un temps d'attente d'au moins deux heures, de telle manière que l'on obtienne un récipient de transport et de transfert (110) sans bulle de gaz.

3. Procédé de fabrication selon l'une quelconque des revendications précédentes, dans lequel à l'étape de procédé d) on contraint au moins un élément de ressort (136) du récipient de transport et de transfert (110), dans lequel l'élément de ressort (136) agit sur au moins une partie déplaçable du réservoir (114), en particulier sur au moins un bouchon (116) et de préférence encore sur au moins un bouchon perforable (116).

4. Procédé de fabrication selon l'une quelconque des revendications précédentes, dans lequel le réservoir (114) est, immédiatement après l'exécution de l'étape de procédé d), sans communication fluidique entre un espace intérieur (120) du réservoir (114) contenant le milieu fluide (112) et un environnement (148) du réservoir (114), dans lequel le réservoir (114) présente au moins une partie perforable (118), à travers laquelle un prélèvement du milieu fluide (112) peut être effectué après une perforation de la partie perforable (118).

5. Procédé de fabrication selon l'une quelconque des revendications précédentes, dans lequel l'élément de ressort (136) presse, après l'exécution de l'étape de procédé d), au moins une partie du réservoir (114) contre au moins un contre-appui (142) du récipient de transport et de transfert (110), dans lequel le contre-appui (142) comprend au moins une ouverture de perforation (146), dans lequel au moins un élément de perforation (152) peut pénétrer à travers l'ouverture de perforation (146) jusqu'à au moins une partie perforable (118) du réservoir (114).

6. Procédé de préparation pour la préparation d'au moins un milieu fluide (112), comprenant:
- une fabrication d'un récipient de transport et de transfert (110) en utilisant un procédé de fabrication selon l'une quelconque des revendications précédentes; et
- une perforation d'au moins une partie perforable (118) pour la création d'une communication fluidique entre un espace intérieur (120) du réservoir (114) et un espace extérieur en vue d'un prélèvement du milieu fluide (112) hors du réservoir (114).

7. Procédé de préparation selon la revendication précédente, comprenant en outre au moins une étape d'interruption, dans lequel dans l'étape d'interruption on arrête la perforation de la partie perforable (118) et on interrompt la communication fluidique.

8. Récipient de transport et de transfert (110) destiné à stocker et préparer au moins un milieu fluide (112), en particulier pour le stockage et la préparation d'au moins un liquide stérile, dans lequel le récipient de transport et de transfert (110) comprend:
A) au moins un réservoir (114) avec le milieu fluide (112) qu'il contient, dans lequel le réservoir (114) présente une paroi de réservoir (115) et au moins une partie perforable (118) sous la forme d'un bouchon déplaçable (116), dans lequel au moyen d'une perforation de la partie perforable (118) une communication fluidique jusqu'à un espace intérieur (120) du réservoir (114) peut être créée en vue d'un prélèvement du milieu fluide (112) hors du réservoir (114);
B) au moins une source d'énergie intégrée (134), dans lequel la source d'énergie (134) est conçue pour provoquer une vidange du milieu fluide (112) hors du réservoir (114) lors d'une utilisation du récipient de transport et de transfert (110), en particulier une vidange complète, dans lequel la source d'énergie (134) comprend au moins un élément de ressort (136), dans lequel la source d'énergie (134) est conçue pour soumettre le milieu fluide (112) dans le réservoir (114) à une surpression, dans lequel la surpression peut être maintenue jusqu'à un prélèvement du milieu fluide (112),
dans lequel le récipient de transport et de transfert (110) présente en outre au moins un emballage (151), dans lequel au moins le réservoir (114) est contenu dans l'emballage (151) dans l'état soumis à la surpression.

9. Récipient de transport et de transfert (110) selon la revendication précédente, comprenant en outre au moins un réservoir extérieur (126), dans lequel le réservoir (114) est contenu au moins en partie dans le réservoir extérieur (126), dans lequel le réservoir extérieur (126) et la source d'énergie (134) sont conçus de telle manière que la soumission du milieu fluide (112) à la pression soit effectuée pendant une communication du réservoir extérieur (126) avec le réservoir (114) et/ou pendant une fermeture du réservoir extérieur (126), dans lequel la source d'énergie (134) est de préférence contrainte mécaniquement.

10. Récipient de transport et de transfert (110) selon une des revendications précédentes concernant un récipient de transport et de transfert (110), dans lequel l'élément de ressort (136) comprend en particulier au moins un ressort de pression hélicoïdal, dans lequel l'élément de ressort (136) est conçu pour presser au moins une partie du réservoir (114), en particulier la partie perforable (118), et de préférence encore un bouchon perforable (116), contre au moins un contre-appui (142) du récipient de transport et de transfert (110).

11. Récipient de transport et de transfert (110) selon une des revendications précédentes concernant un récipient de transport et de transfert (110), dans lequel le récipient de transport et de transfert (110) comprend au moins un élément de perforation (152) pour la perforation de la partie perforable (118).

12. Récipient de transport et de transfert (110) selon une des revendications précédentes concernant un récipient de transport et de transfert (110), comprenant en outre au moins un élément d'injection (156), dans lequel l'élément d'injection (156) est conçu pour être piqué dans un tissu corporel humain ou animal et/ou dans un récipient (158) réalisé séparément du réservoir (114).

13. Récipient de transport et de transfert (110) selon la revendication précédente, dans lequel le récipient de transport et de transfert (110) est configuré de telle manière qu'une création d'une communication fluidique entre l'élément d'injection (156) et l'espace intérieur (120) et la piqûre de l'élément d'injection (156) dans le tissu corporel humain ou animal et/ou dans le récipient (158) réalisé séparément du réservoir (114) forment des opérations séparées.

14. Récipient de transport et de transfert (110) selon une des revendications précédentes concernant un récipient de transport et de transfert (110), dans lequel le récipient de transport et de transfert (110) présente au moins un élément de commutation (166), dans lequel l'élément de commutation (166) est conçu pour, dans au moins une première position de commutateur, maintenir au moyen d'une perforation de la partie perforable (118) la communication fluidique jusqu'à l'espace intérieur (120) du réservoir (114), dans lequel l'élément de commutation est en outre conçu pour, dans au moins une deuxième position de commutateur, interrompre la communication fluidique, de préférence en interrompant la perforation de la partie perforable (118).

15. Récipient de transport et de transfert (110) selon une des revendications précédentes concernant un récipient de transport et de transfert (110), dans lequel la source d'énergie (134) est conçue pour agir mécaniquement sur la partie perforable (118).
